# EUROPEAN PATENT APPLICATION

(11) **EP 4 709 126 A2**
(43) Date of publication of application: **11.03.2026**
(21) Application number: 26153109.9
(22) Date of filing: 11.12.2023
(51) Int. Cl.: H10K 85/60

(54) **COMPOUND, PHOTOELECTRIC CONVERSION ELEMENT MATERIAL, AND PHOTOELECTRIC CONVERSION ELEMENT**

(30) Priority: 09.12.2022 JP 2022197533
(62) Divisional of application: 23900761.0
(71) Applicant: Tosoh Corporation, Shunan-shi, Yamaguchi 746-8501 (JP)
(72) Inventor: OIKE, Kana, Ayase-shi, Kanagawa 252-1123 (JP); MORINAKA, Yuta, Ayase-shi, Kanagawa 252-1123 (JP); ONO, Yohei, Ayase-shi, Kanagawa 252-1123 (JP)
(74) Representative: TBK

(57) **Abstract**

Provided are a compound, a photoelectric conversion element material and a photoelectric conversion element which are excellent in response speed and exhibit a high external quantum yield, and a photoelectric conversion element material that contributes to production of these elements.

## Description

### Technical Field

The present invention relates to a photoelectric conversion element, an imaging element, a photoelectric conversion element material, and a compound.

### Background Art

Photoelectric conversion elements are widely utilized in solar cells, photosensors, image sensors, and the like. The application and market of photoelectric conversion elements are expanding, and development thereof is being carried out vigorously.

For example, Patent Literature 1 discloses a photoelectric conversion element which includes a hole blocking layer containing a pyrimidine derivative.

For example, Patent Literature 2 discloses a photoelectric conversion element which includes a hole blocking layer containing a triazine derivative.

### Citation List

### [Patent Literature]

[Patent Literature 1]
   Japanese Patent Application Publication Tokukai No. 2022-17302
[Patent Literature 2]
   Korean Patent Publication No. 10-2021-053141

### Summary of Invention

### Technical Problem

For example, for an imaging element which is a photoelectric conversion element, an element is demanded which is excellent in both response speed and external quantum efficiency.

An object of the invention in accordance with an aspect of the present invention is to provide a photoelectric conversion element and an imaging element which are excellent in response speed and exhibit a high external quantum yield, and a photoelectric conversion element material and a hole blocking material which are used in the photoelectric conversion element and the imaging element.

### Solution to Problem

According to an aspect of the present invention, a photoelectric conversion element is provided which includes a layer containing a photoelectric conversion element material represented by formula (1) below.

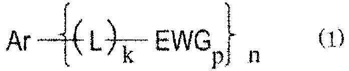

In the formula (1),
Ar represents a condensed ring aromatic hydrocarbon group having 16 to 40 carbon atoms,
EWG represents an electron-withdrawing group,
L represents an aromatic hydrocarbon group having 6 to 30 carbon atoms,
n represents an integer of 1 to 8,
k represents an integer of 0 to 2,
p represents an integer of 1 to 8,
in a case where k is 0, p is 1,
in a case where there are two or more EWG, the two or more EWG are identical or different electron-withdrawing groups, and
each of Ar, L, and EWG has at least one substituent or has no substituent.

According to another aspect of the present invention, the photoelectric conversion element in accordance with the above aspect is an imaging element.

According to another aspect of the present invention, provided is a photoelectric conversion element in which the layer containing the photoelectric conversion element material is a hole blocking layer.

According to another aspect of the present invention, provided is a photoelectric conversion element material for forming the layer which is included in the photoelectric conversion element in accordance with the above aspect.

According to another aspect of the present invention, provided is a photoelectric conversion element material in which the photoelectric conversion element material in accordance with the above aspect is a photoelectric conversion element material for an imaging element.

According to another aspect of the present invention, provided is the photoelectric conversion element material in accordance with the above aspect which is a hole blocking material.

According to another aspect of the present invention, a compound is provided which is represented by formula (3) below: 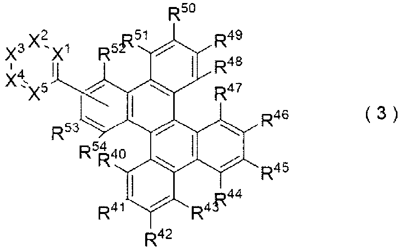
X¹ through X⁵ each represent a nitrogen atom or CR⁵⁵,
the number of nitrogen atoms in X¹ through X⁵ is an integer of 0 to 3,
R⁴⁰ through R⁵⁵ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R⁴⁰ through R⁵⁵ form a ring by bonding of two adjacent groups thereof,
at least one R⁵⁵ is selected from a cyano group, a halogen atom, and an alkyl halide group,
in a case where X¹ through X⁵ are all CR⁵⁵, two or more of R⁵⁵ are selected from a cyano group, a halogen atom, and an alkyl halide group, and
R⁴⁰ through R⁵⁵ each independently have further at least one substituent or have no substituent.

According to another aspect of the present invention, a compound is provided which is represented by formula (4-1) below: 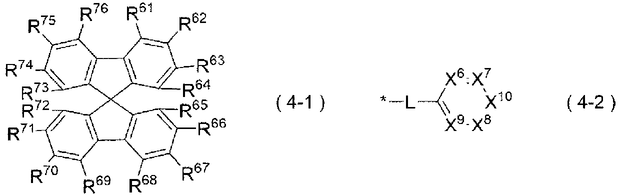 where
R⁶¹ through R⁷⁶ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, a heteroaryl group having 3 to 30 carbon atoms, and a group represented by formula (4-2), or form a ring by bonding of two adjacent groups thereof,
one of R⁶¹ through R⁶⁴ is a group represented by formula (4-2),
L represents a direct bond or an aromatic hydrocarbon group having 6 to 16 carbon atoms,
X⁶ through X¹⁰ each represent a nitrogen atom, C(CN), or CR⁷⁷,
among X⁹ and X¹⁰, X⁹ is C(CN) or X¹⁰ is a nitrogen atom or C(CN),
among X⁶ through X¹⁰, the number of nitrogen atoms is 0 or 1, the number of C(CN) is 2 or 3 in a case where the number of nitrogen atoms is 0, and the number of C(CN) is 2 in a case where the number of nitrogen atoms is 1,
each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, and an alkoxy group having 1 to 10 carbon atoms, and
R⁶¹ through R⁷⁷ and L have at least one substituent or have no substituent.

### Advantageous Effects of Invention

According to an aspect of the present invention, it is possible to provide a photoelectric conversion element and an imaging element which are excellent in response speed and exhibit a high external quantum yield, and a photoelectric conversion element material and a hole blocking material which are used in the photoelectric conversion element and the imaging element.

### Brief Description of Drawings

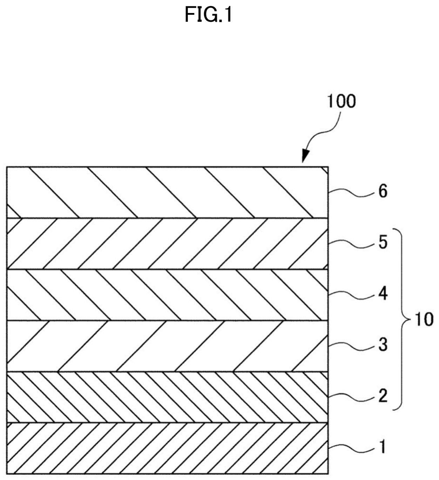

Fig. 1 is a cross-sectional view schematically illustrating a laminated structure of a photoelectric conversion element for an imaging element including a photoelectric conversion element material in accordance with an aspect of the present invention.

### Description of Embodiments

The following description will discuss, in detail, a photoelectric conversion element material which is contained in a layer provided in a photoelectric conversion element in accordance with an aspect of the present invention.

### <Photoelectric conversion element material (1)>

A photoelectric conversion element in accordance with an aspect of the present invention includes a layer of a photoelectric conversion element material which is represented by formula (1) below.

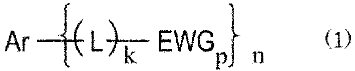

In formula (1),
Ar represents a condensed ring aromatic hydrocarbon group having 16 to 40 carbon atoms,
EWG represents an electron-withdrawing group,
L represents an aromatic hydrocarbon group having 6 to 30 carbon atoms,
n represents an integer of 1 to 8,
k represents an integer of 0 to 2,
p represents an integer of 1 to 8,
in a case where k is 0, p is 1,
in a case where there are two or more EWG, the two or more EWG are identical or different electron-withdrawing groups, and
each of Ar, L, and EWG has at least one substituent or has no substituent.

That is, in this specification, a group that is represented by formula (1) and that is not a group represented by formula (1') below which is included in the photoelectric conversion element material is described as a "substituent".

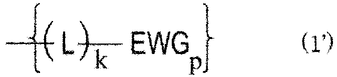

Definitions of L, EWG, k, and p in formula (1') are identical with those of L, EWG, k, and p in formula (1).

The compound represented by formula (1) is (i) an electron transport material which is excellent in electric charge, i.e., electron transport property and (ii) a hole blocking material which is excellent in reverse electric charge, i.e., hole blocking property. The compound can be suitably used as a photoelectric conversion element material.

Definitions of groups in formula (1) and formula (1') and preferable specific examples thereof are as follows.

### <Regarding Ar>

Ar is a condensed ring aromatic hydrocarbon group in which an aromatic ring is condensed, and the condensed ring aromatic hydrocarbon group may have a substituent. The condensed ring aromatic hydrocarbon group which is Ar has carbon atoms whose number falls within a range of 16 to 40, preferably a range of 20 to 40. In the range of 16 to 40 carbon atoms in Ar, higher thermal stability can be imparted to the photoelectric conversion element material as the number of carbon atoms increases, and it is possible, as the number of carbon atoms decreases, to prevent a LUMO level of the photoelectric conversion element material from becoming excessively low.

Ar is preferably, for example, a condensed ring aromatic hydrocarbon group which encompasses four or more 6-membered rings. In a case where Ar is a condensed ring aromatic hydrocarbon group in which four or more 6-membered rings are condensed, it is possible to heighten Tg of the photoelectric conversion element material, and it is thus possible to enhance thermal stability of the photoelectric conversion element material.

The following indicates preferable specific examples of Ar. Note, however, that Ar is not limited to those specific examples.

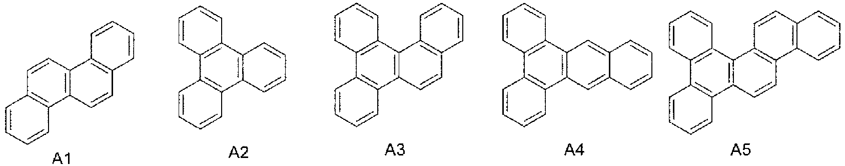

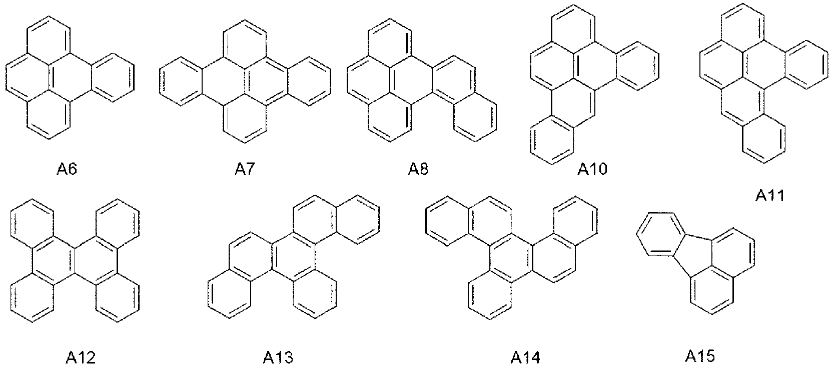

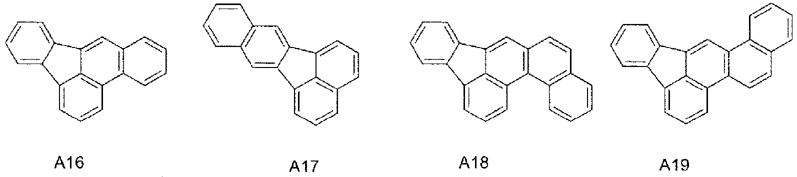

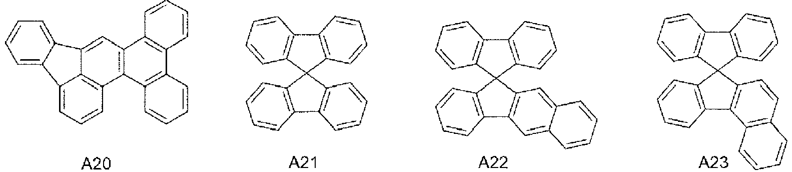

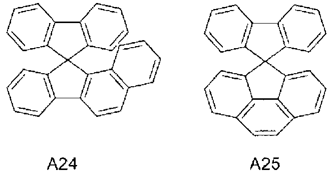

Among those Ar, for example, it is more preferable to employ a condensed ring aromatic hydrocarbon group which is selected from (i) a condensed ring aromatic hydrocarbon group that is selected from a triphenylene group (A2), a fluoranthene group (A15), and a spirofluoren group (A21), (ii) condensed ring aromatic hydrocarbon groups (A3) through (A14) encompassing a triphenylene group, (iii) condensed ring aromatic hydrocarbon groups (A16) through (A20) encompassing a fluoranthene group, and (iv) condensed ring aromatic hydrocarbon groups (A22) through (A25) encompassing a spirofluoren group.

Ar may have a substituent. For example, Ar may have a structure represented by formulae (2-1), (2-2), and (2-3) below.

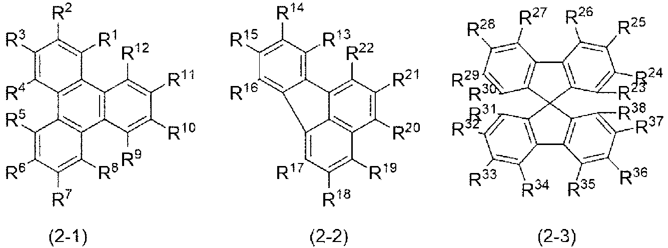

In the formula (2-1), formula (2-2), and formula (2-3),
R¹ through R³⁸ are preferably each independently at least one substituent selected from a hydrogen atom, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms.

The aromatic hydrocarbon group having 6 to 30 carbon atoms is identical with groups described later as groups represented by L. The heteroaryl group having 3 to 30 carbon atoms is preferably selected from heteroaryl groups that each have 3 to 30 carbon atoms and contain a nitrogen atom and are represented by EWG described later.

Some of R¹ through R³⁸ may form, as a substituent, a ring by bonding of two adjacent groups thereof, and the ring may be a hydrocarbon ring. More specifically, two adjacent groups among R¹ through R¹² represented by formula (2-1) may be bonded together to form a ring. Thus, it is possible to include a structure represented by formula (2-1), for example, a condensed ring aromatic hydrocarbon group selected from the condensed ring aromatic hydrocarbon groups (A4) through (A14) described above. Two adjacent groups among R¹² through R²² represented by formula (2-2) may be bonded together to form a ring. Thus, it is possible to include a structure represented by formula (2-2), for example, a condensed ring aromatic hydrocarbon group selected from the condensed ring aromatic hydrocarbon groups (A16) through (A20) described above. Two adjacent groups among R²³ through R³⁸ represented by formula (2-3) may be bonded together to form a ring. Thus, it is possible to include a structure represented by formula (2-3), for example, a condensed ring aromatic hydrocarbon group selected from the condensed ring aromatic hydrocarbon groups (A22) through (A25) described above.

For Ar represented by formulae (2-1), (2-2), or (2-3), some of R¹ through R³⁸ may each be substituted by a group represented by formula (1') or a hydrogen atom included in R¹ through R³⁸ may be substituted by a group represented by formula (1').

R¹ through R³⁵ may each independently have further at least one substituent or have no substituent.

### <Regarding EWG>

EWG represents an electron-withdrawing group. In a case where the photoelectric conversion element material of formula (1) includes a plurality of EWG, these EWG may be identical electron-withdrawing groups or may be different electron-withdrawing groups.

The electron-withdrawing group is preferably at least one group which is selected from a cyano group, a nitro group, a halogen atom, an alkyl halide group, an acyl group, a sulfonyl group, a phosphoryl group, and a heteroaryl group that has 3 to 30 carbon atoms and includes a nitrogen atom. The heteroaryl group that has 3 to 30 carbon atoms and includes a nitrogen atom may have at least one substituent or may have no substituent.

The halogen atom represented by EWG is preferably a fluorine atom.

The alkyl halide group represented by EWG is preferably an alkyl halide group having 1 to 5 carbon atoms, more preferably an alkyl halide group having 1 to 3 carbon atoms. The alkyl halide group is preferably a fluorinated alkyl group, preferably a perfluoroalkyl group. The fluorinated alkyl group is preferably a perfluorobutyl group, a perfluoropropyl group, a perfluoroethyl group, or a trifluoromethyl group, more preferably a trifluoromethyl group.

Examples of the acyl group represented by EWG include an alkyl acyl group, an aryl acyl group, and the like, and are preferably an alkyl acyl group having 2 to 5 carbon atoms, and an aryl acyl group having 7 to 15 carbon atoms. Examples of the alkyl acyl group include an acetyl group, a propionyl group, a trifluoroacetyl group, and the like. Examples of the aryl acyl group include a benzoyl group, a cyanobenzoyl group, a fluorobenzoyl group, a trifluoromethylbenzoyl group, and the like.

Examples of the sulfonyl group represented by EWG include an alkylsulfonyl group, an arylsulfonyl group, and the like, and an alkylsulfonyl group having 1 to 5 carbon atoms and an arylsulfonyl group having 6 to 15 carbon atoms are preferable. Examples of the alkylsulfonyl group include a methylsulfonyl group, an ethylsulfonyl group, an n-propylsulfonyl group, an isopropylsulfonyl group, and the like. Examples of the arylsulfonyl group include a phenylsulfonyl group, a cyanophenylsulfonyl group, a fluorophenylsulfonyl group, and the like.

Examples of the phosphoryl group represented by EWG include a dialkylphosphoryl group, a diarylphosphoryl group, and the like, and a dialkylphosphoryl group having 2 to 10 carbon atoms and a diarylphosphonyl group having 12 to 30 carbon atoms are preferable. Examples of the dialkylphosphoryl group include a dimethylphosphoryl group, a diethylphosphoryl group, a dipropylphosphoryl group, and the like. Examples of the diarylphosphoryl group include a diphenylphosphoryl group, a di(fluorophenyl)phosphoryl group, and the like.

Examples of the heteroaryl group that has 3 to 30 carbon atoms and includes a nitrogen atom include a pyridyl group, a pyrimidyl group, a pyrazyl group, a triazyl group, an imidazolyl group, a quinolyl group, an isoquinolyl group, an azaanthryl group, a diazaanthryl group, a triazaanthryl group, a tetraazaanthryl group, an azaphenanthryl group, a diazaphenanthryl group, a triazaphenanthryl group, a tetraazaphenanthryl group, an azapyrenyl group, a diazapyrenyl group, a triazapyrenyl group, a tetraazapyrenyl group, an azafluoranthenyl group, a diazafluoranthenyl group, a triazafluoranthenyl group, a tetraazafluoranthenyl group, an azatriphenylenyl group, a diazatriphenylenyl group, a triazatriphenylenyl group, a tetraazatriphenylenyl group, a pentaazatriphenylenyl group, a hexaazatriphenylenyl group, an oxazolyl group, a pyrrolyl group, an imidazolyl group, a triazolyl group, a thiadiazolyl group, an oxadiazolyl group, a benzothiazolyl group, a benzoxazolyl group, a benzothiadiazolyl group, and a benzoxadiazolyl group.

Examples of the substituent of the heteroaryl group that has 3 to 30 carbon atoms and includes a nitrogen atom, that is, the substituent of EWG include a deuterium atom, a cyano group, a halogen atom, an alkyl halide group, an acyl group, a nitro group, a sulfonyl group, a phosphoryl group, an alkyl group having 1 to 20 carbon atoms, an alkenyl group and a cycloalkyl group, an alkoxy group having 1 to 10 carbon atoms, a group represented by -P(=O)(Ar')₂, a group represented by - OSO₂Ar', a group represented by -S(=O)Ar', a group represented by -B(Ar')₂, a group represented by -B(OAr')₂, a group represented by -Si(Ar')₃, an aromatic hydrocarbon group having 6 to 30 carbon atoms, and a heteroaryl group having 3 to 30 carbon atoms (Ar' represents an aryl group). Among these substituents of EWG, a cyano group, a halogen atom, an alkyl halide group, an acyl group, a sulfonyl group, a phosphoryl group, an aromatic hydrocarbon group having 6 to 30 carbon atoms, and a heteroaryl group having 3 to 30 carbon atoms are preferable, a cyano group, a halogen atom, an alkyl halide group, a heteroaryl group having 3 to 30 carbon atoms are more preferable, a cyano group, a fluorine atom, and a fluorinated alkyl group are further preferable, a cyano group, a fluorine atom, and a trifluoromethyl group are particularly preferable. The alkyl halide group, acyl group, sulfonyl group, phosphoryl group, and heteroaryl group having 3 to 30 carbon atoms in the substituents of EWG are identical with those described as groups represented by EWG. The aromatic hydrocarbon group having 6 to 30 carbon atoms is identical with those described later as groups represented by L.

EWG is preferably a cyano group, a fluorine atom, a fluoroalkyl group, and a heteroaryl group that has 3 to 30 carbon atoms and includes a nitrogen atom, more preferably a cyano group, a fluorine atom, a fluoroalkyl group, and a heteroaryl group that has 3 to 30 carbon atoms, includes a nitrogen atom, and has, as a substituent of EWG, at least one group selected from a cyano group, a fluorine atom, and a fluoroalkyl group.

### <Regarding L>

L represents an aromatic hydrocarbon group having 6 to 30 carbon atoms, and the aromatic hydrocarbon group having 6 to 30 carbon atoms may have a substituent.

Examples of the aromatic hydrocarbon group having 6 to 30 carbon atoms include a phenyl group, a naphthyl group, a phenanthryl group, an anthryl group, a fluorenyl group, a dimethylfluorenyl group, a spirofluorenyl group, a pyrenyl group, a fluoranthenyl group, a triphenylenyl group, a tetracenyl group, a chrysenyl group, and the like. Among these, a phenyl group and a naphthyl group are preferable. Substituents which are included in the aromatic hydrocarbon groups having 6 to 30 carbon atoms are identical with those described as substituents of EWG such as a heteroaryl group that has 3 to 30 carbon atoms and includes a nitrogen atom, and therefore a description thereof is omitted.

### <Regarding n, k and p>

n preferably represents an integer of 1 to 8. In the photoelectric conversion element material represented by formula (1), in a case where n is optimized within a range of 1 to 8, it is possible to inhibit a decrease in Tg of the photoelectric conversion element material, to deepen a LUMO level of the photoelectric conversion element material, and to heighten a response speed of a photoelectric conversion element.

k is represented by an integer of 0 to 2 and is preferably an integer of 0 or 1. In a case where k is 1, it is more preferable that EWG is a cyano group, a fluorine atom, or a fluoroalkyl group. Thus, it is possible to deepen a LUMO level of the photoelectric conversion element material while inhibiting a decrease in Tg of the photoelectric conversion element material, and to heighten a response speed of a photoelectric conversion element.

p represents an integer of 1 to 8. In a case where k is 0, p may be 1.

The photoelectric conversion element material represented by formula (1) above heightens a glass transition temperature Tg as described later by the above Ar structure. This prevents decreases in external quantum efficiency and dark current in a photoelectric conversion element including a layer which is formed from the photoelectric conversion element material by heating. In the photoelectric conversion element material represented by formula (1) above, by including the above EWG, a response speed with respect to light is enhanced. Thus, the photoelectric conversion element material can be suitably used in a photoelectric conversion element which is demanded to achieve both satisfactory response speed and external quantum efficiency.

The photoelectric conversion element material which is included in the photoelectric conversion element in accordance with an aspect of the present invention is not limited to the above aspect. Ar represented by formula (1) is preferably a condensed ring aromatic hydrocarbon group represented by any of (A12), (A20), (A21), and (A-22) through (A-25), from the viewpoint of further enhancing Tg of a compound including the condensed ring aromatic hydrocarbon group. More preferably, the compound in accordance with an aspect of the present invention can be a compound which includes a condensed ring aromatic hydrocarbon group and which is selected from compounds represented by formula (3) below and formula (4-1) and formula (4-2) below.

### <Compound (photoelectric conversion element material (2))>

The photoelectric conversion element material which is included in the photoelectric conversion element in accordance with an aspect of the present invention is not limited to the above aspect. For example, the following compound represented by formula (3) below is encompassed in an aspect of the present invention.

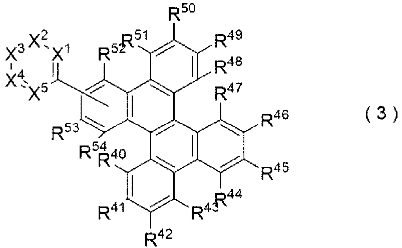

In the formula (3),
X¹ through X⁵ each represent a nitrogen atom or CR⁵⁵,
the number of nitrogen atoms in X¹ through X⁵ is an integer of 0 to 3,
R⁴⁰ through R⁵⁵ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R⁴⁰ through R⁵⁵ form a ring by bonding of two adjacent groups thereof,
at least one R⁵⁵ is selected from a cyano group, a halogen atom, and an alkyl halide group,
in a case where X¹ through X⁵ are all CR⁵⁵, two or more of R⁵⁵ are selected from a cyano group, a halogen atom, and an alkyl halide group, and
R⁴⁰ through R⁵⁵ each independently have further at least one substituent or have no substituent.

The compound represented by formula (3) includes a condensed ring aromatic hydrocarbon group, and the condensed ring aromatic hydrocarbon group can be (A12) which is exemplified as Ar represented by formula (1).

### <Regarding X¹ through X⁵>

X¹ through X⁵ each represent a nitrogen atom or CR⁵⁵. Among X¹ through X⁵, the number of nitrogen atoms is an integer of 0 to 3.

It is preferable that X¹ through X⁵ are all CR⁵⁵ or one of X² through X⁴ is a nitrogen atom.

### <Regarding R⁴⁰ through R⁵⁵>

Substituents represented by R⁴⁰ through R⁵⁵ and preferable specific examples thereof are identical with examples of the substituent represented by formula (1). Further preferable specific examples of substituents included in R⁴⁰ through R⁵⁵ are identical with substituents of EWG represented by formula (1).

### <Regarding R⁵⁵>

R⁵⁵ may be selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms.

One of R⁵⁵ may be a cyano group, a halogen atom, or an alkyl halide group, and two adjacent R⁵⁵ may be bonded together to form a ring.

In the compound represented by formula (3), the halogen atom is a fluorine atom, and the aromatic hydrocarbon group having 6 to 30 carbon atoms may be substituted or may not be substituted by a group selected from a cyano group, a fluorine group, and a fluoroalkyl group.

### <Compound (photoelectric conversion element material (3))>

The photoelectric conversion element material which is included in the photoelectric conversion element in accordance with an aspect of the present invention is not limited to the above aspect. For example, the following compound represented by formula (4-1) below is encompassed in an aspect of the present invention.

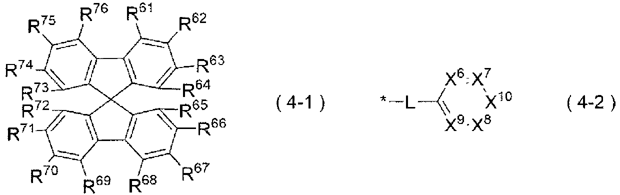

In the formula (4-1),
R⁶¹ through R⁷⁶ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, a heteroaryl group having 3 to 30 carbon atoms, and a group represented by formula (4-2), or form a ring by bonding of two adjacent groups thereof,
one of R⁶¹ through R⁶⁴ is a group represented by formula (4-2),
L represents a direct bond or an aromatic hydrocarbon group having 6 to 16 carbon atoms,
X⁶ through X¹⁰ each represent a nitrogen atom, C(CN), or CR⁷⁷,
among X⁹ and X¹⁰, X⁹ is C(CN) or X¹⁰ is a nitrogen atom or C(CN),
among X⁶ through X¹⁰, the number of nitrogen atoms is 0 or 1, the number of C(CN) is 2 or 3 in a case where the number of nitrogen atoms is 0, and the number of C(CN) is 2 in a case where the number of nitrogen atoms is 1, and
each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, and an alkoxy group having 1 to 10 carbon atoms.

### <Regarding R⁶¹ through R⁷⁶>

Except for a case of being selected from groups represented by formula (4-2), R⁶¹ through R⁷⁶ in formula (4-1) can each be selected from groups similar to those of R⁴⁰ through R⁵⁵ in the compound represented by formula (3). Here, in formula (4-1), R⁶¹ through R⁷⁶ (a) can each be selected from a cyano group, a nitro group, a halogen atom, an alkyl halide group, an acyl group, a sulfonyl group, a phosphoryl group, a heteroaryl group having 3 to 30 carbon atoms, and a group represented by formula (4-2) or (b) can each be selected from a hydrogen atom, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, and an aromatic hydrocarbon group having 6 to 30 carbon atoms and can form a ring by bonding of two adjacent groups thereof.

From the viewpoint of deepening a LUMO level of the compound, R⁶¹ through R⁷⁶ in formula (4-1) are each preferably selected from the above described (a), can each be selected from groups represented by EWG in the foregoing formula (1), and are each more preferably selected from a cyano group, a heteroaryl group having 3 to 16 carbon atoms, and a group represented by formula (4-2). Note, however, that, in a case where R⁶¹ through R⁷⁶ are each selected from a heteroaryl group having 3 to 16 carbon atoms, the heteroaryl group may include or may not include a carbazolyl group.

From the viewpoint of heightening a glass transition temperature Tg, R⁶¹ through R⁷⁶ are each preferably selected from the above described (b), more preferably selected from a hydrogen atom, an alkyl group having 1 to 20 carbon atoms, a cycloalkyl group having 3 to 20 carbon atoms, and an aromatic hydrocarbon group having 6 to 16 carbon atoms.

In a case where two adjacent groups of R⁶¹ through R⁷⁶ in formula (4-1) are bonded together to form a ring, the ring can constitute a part of the condensed ring aromatic hydrocarbon group that is exemplified as Ar in formula (1). That is, the compound represented by formula (4-1) includes a condensed ring aromatic hydrocarbon group. The condensed ring aromatic hydrocarbon group can be (A21) exemplified as Ar represented by formula (1). In a case where R⁶¹ through R⁷⁶ form a ring, the condensed ring aromatic hydrocarbon group can be a condensed ring aromatic hydrocarbon group exemplified by (A-22) through (A-25). Forming a ring by R⁶¹ through R⁷⁶ in formula (4-1) is preferable from the viewpoint of making it possible to further heighten Tg of the compound.

In the group represented by formula (4-2), L is selected from aromatic hydrocarbon groups having 6 to 16 carbon atoms among the specific examples of L indicated in formula (1). L in formula (4-2) can be a phenyl group, a naphthyl group, a phenanthryl group, an anthryl group, a fluorenyl group, a dimethylfluorenyl group, or the like. The aromatic hydrocarbon group having 6 to 16 carbon atoms may have a substituent. Here, a substituent included in the aromatic hydrocarbon group having 6 to 16 carbon atoms may be selected from those described as substituents of EWG, such as a heteroaryl group that has 3 to 30 carbon atoms and includes a nitrogen atom. In a case where L indicated in formula (4-2) is a direct bond, such a case of course falls under a case where k of formula (1) is 0.

In the group represented by formula (4-2), a ring structure including X⁶ through X¹⁰ is selected from a phenyl group substituted by two or three nitrile groups and a pyridyl group substituted by two nitrile groups. The ring structure includes R⁷⁷ as a group other than a nitrile group. The ring structure is an electron-withdrawing group, as with EWG represented by formula (1). It is more preferable that, in the ring structure including X⁶ through X¹⁰, X¹⁰ is a nitrogen atom or C(CN), X⁷ and X⁸ are C(CN) or CR⁷⁷, and X⁶ and X⁹ are CR⁷⁷.

Each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, and an alkoxy group having 1 to 10 carbon atoms, and more preferably each independently selected from a hydrogen atom, an alkyl group having 1 to 20 carbon atoms, and a cycloalkyl group having 1 to 20 carbon atoms.

Substituents represented by R⁶¹ through R⁷⁷ have at least one substituent or have no substituent. In a case of having a substituent, the substituent may be selected from substituents described as those represented by EWG such as a heteroaryl group that has 3 to 30 carbon atoms and includes a nitrogen atom.

### <Preferable specific examples of photoelectric conversion element material>

The following indicates preferable specific examples of the photoelectric conversion element material represented by formula (1). Note, however, that the photoelectric conversion element material is not limited to those specific examples.

### [B001- B024]

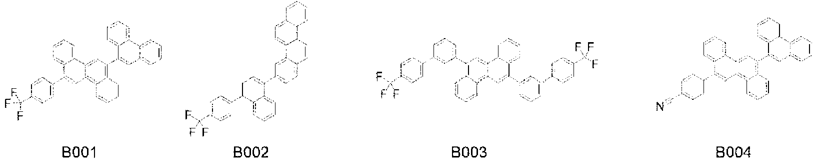

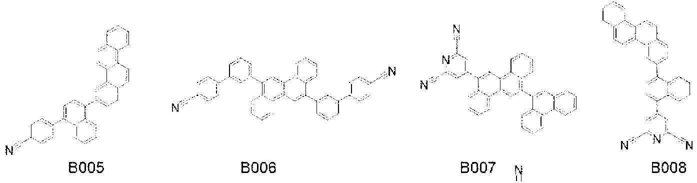

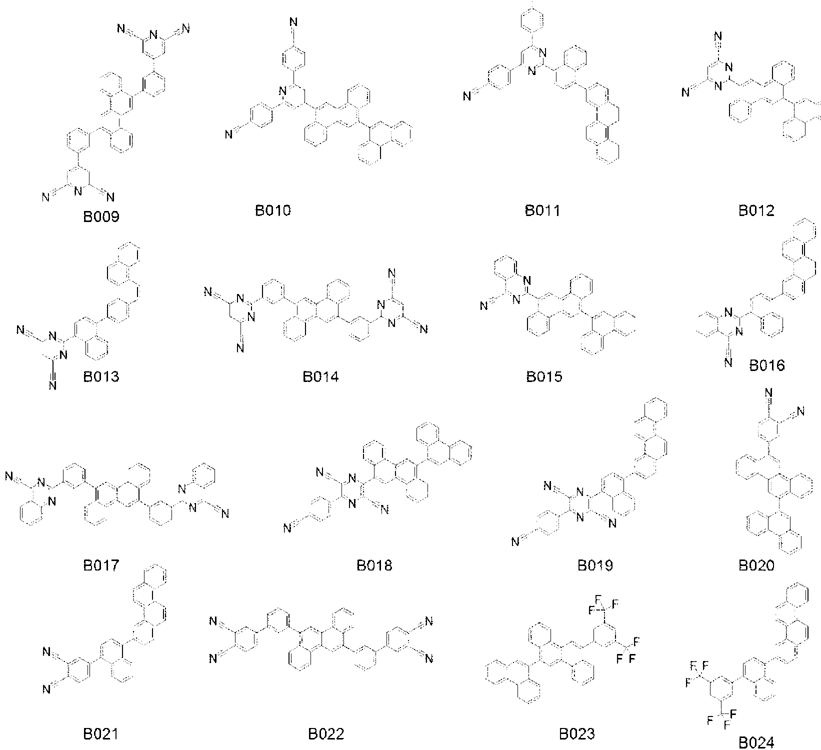

### [B025-B048]

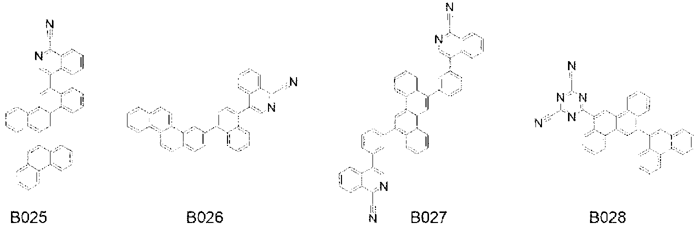

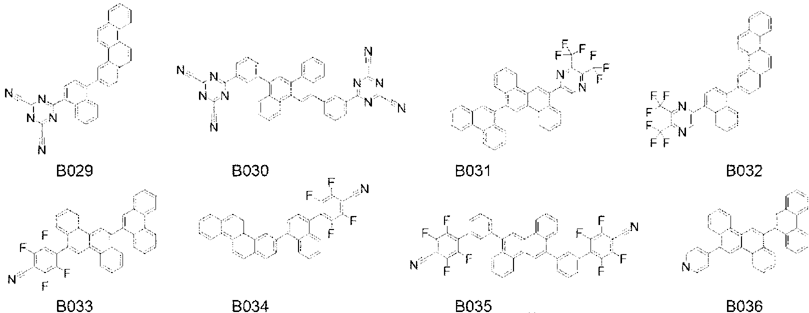

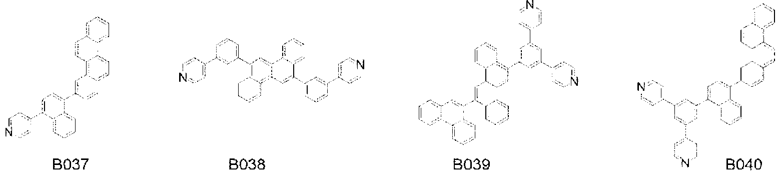

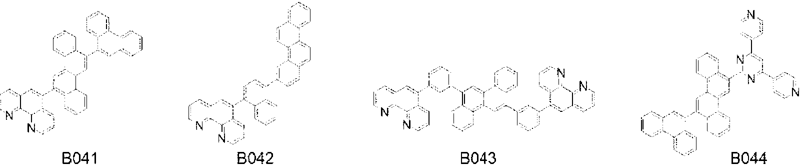

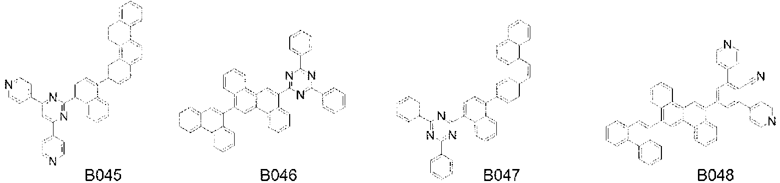

### [B049-B072]

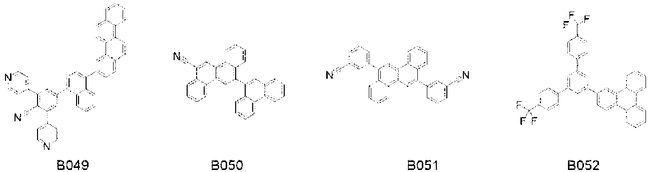

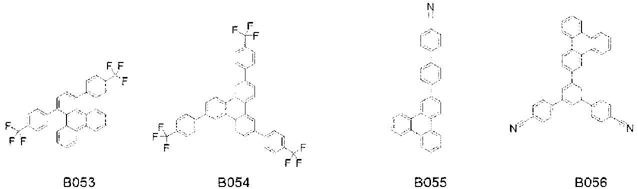

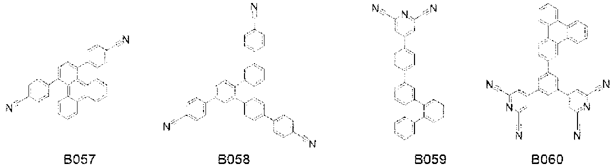

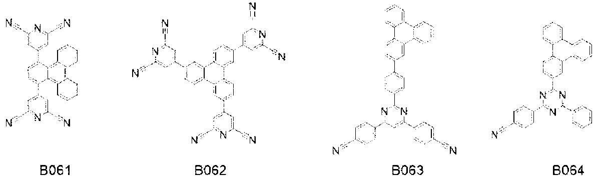

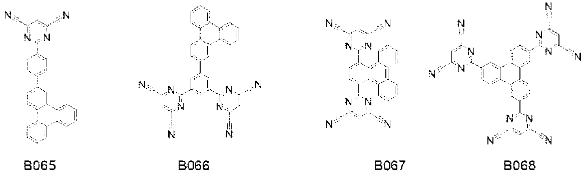

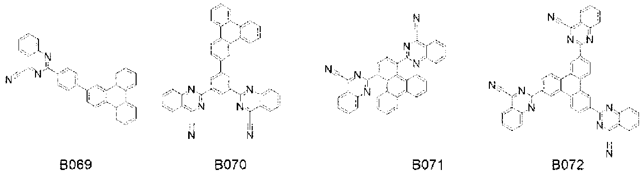

### [B073-B096]

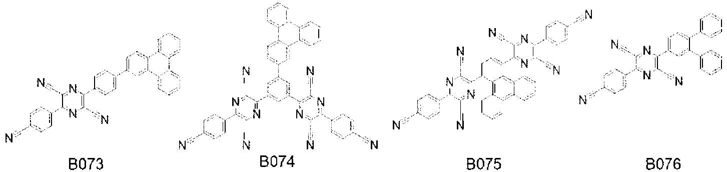

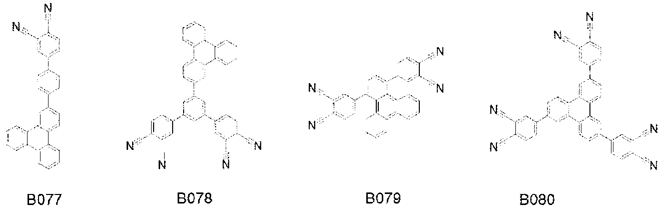

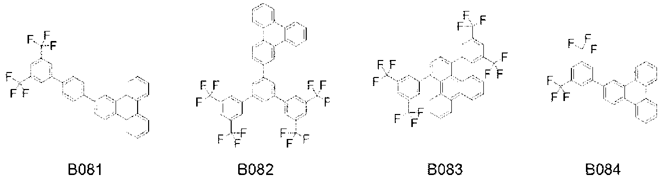

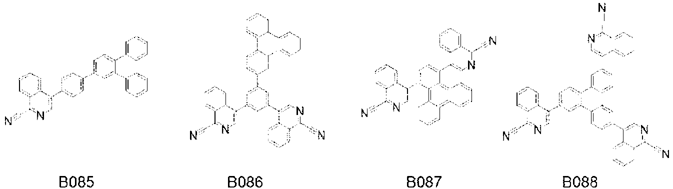

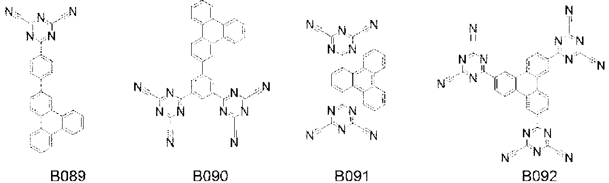

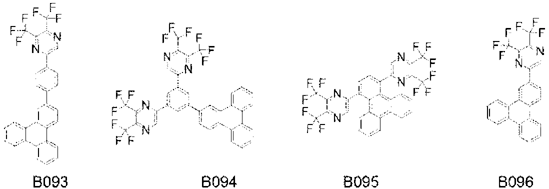

### [B097-B120]

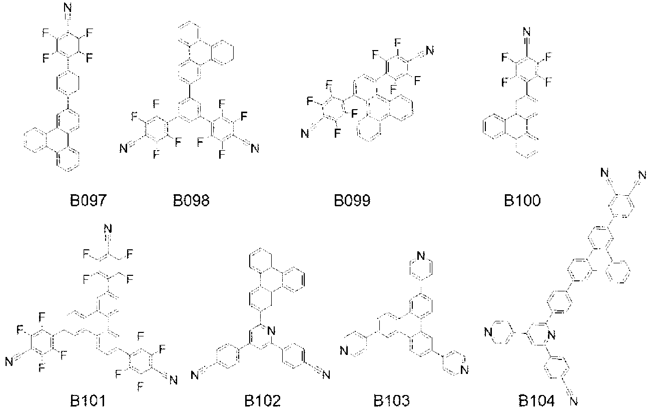

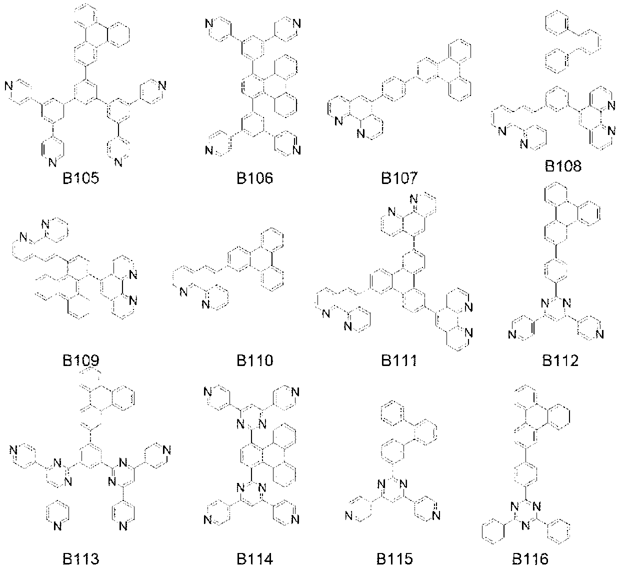

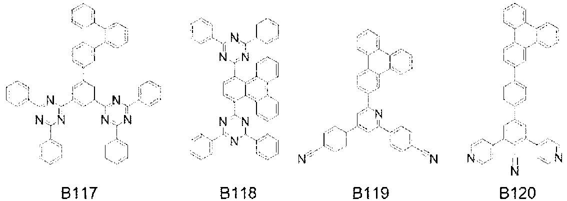

### [B121-B144]

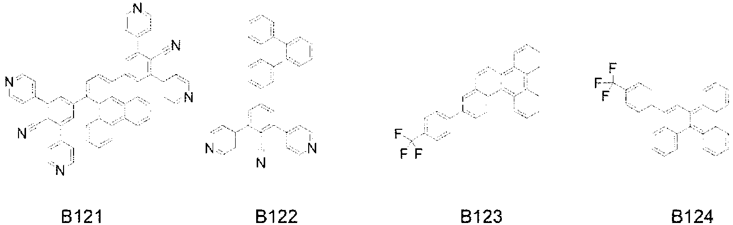

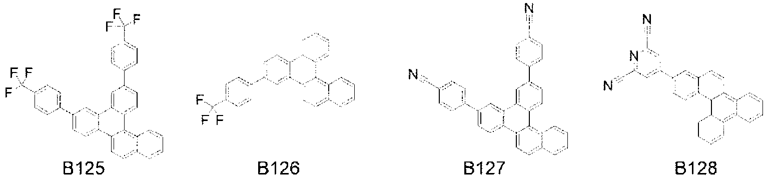

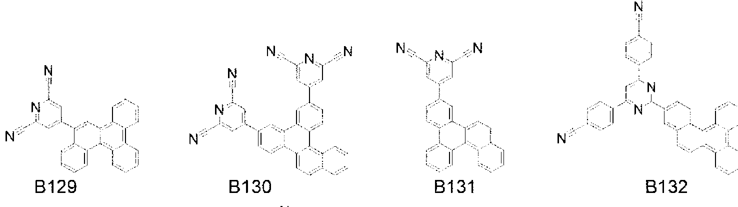

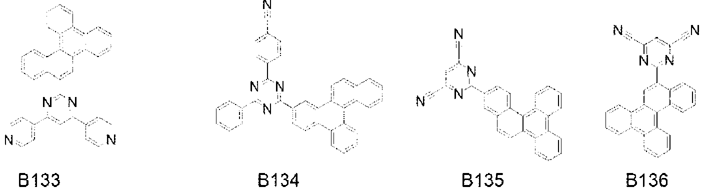

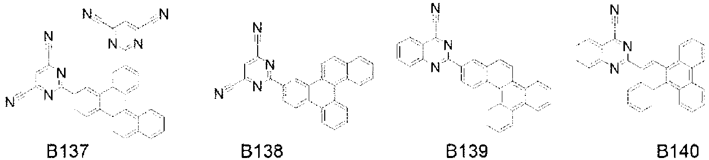

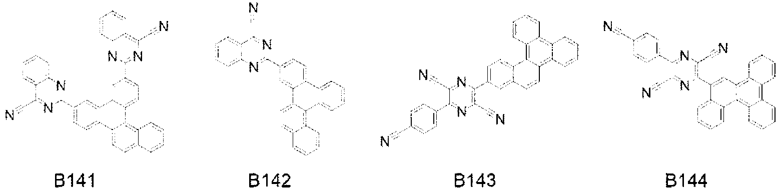

### [B145-B168]

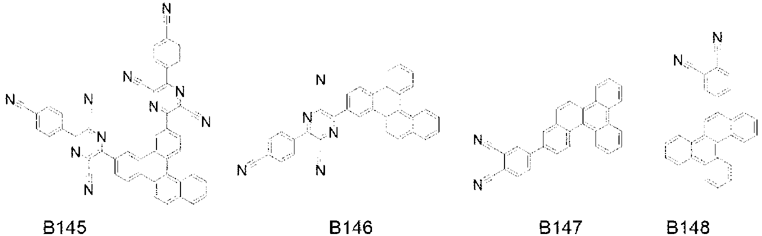

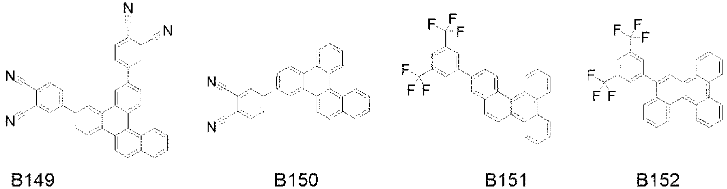

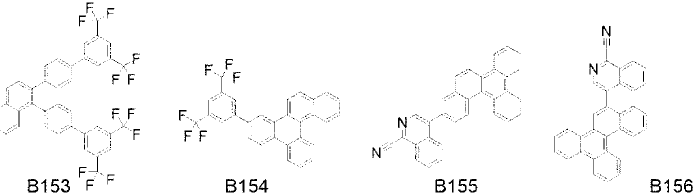

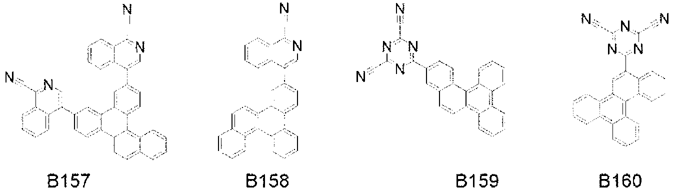

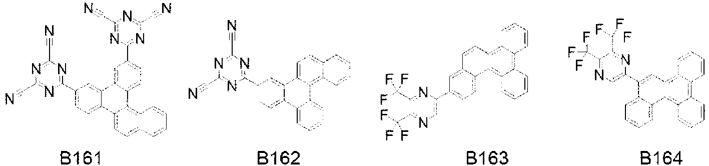

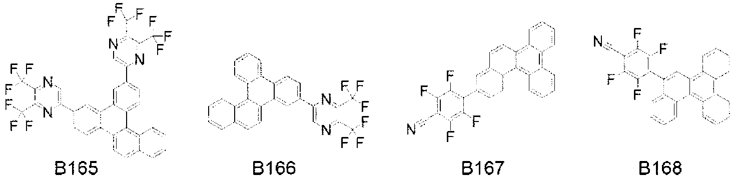

### [B169-B192]

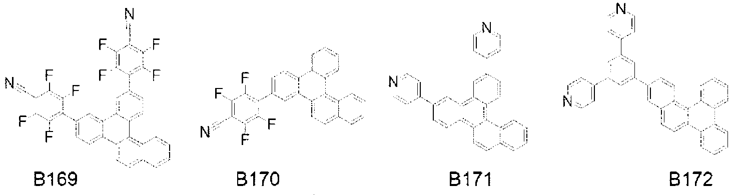

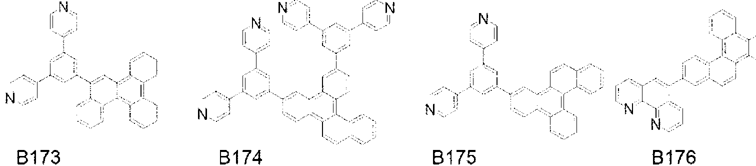

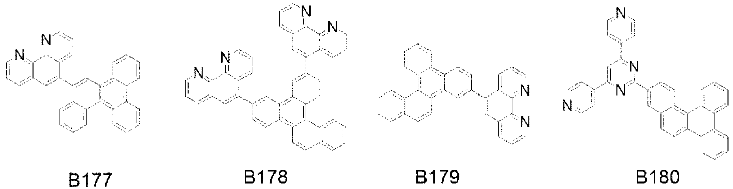

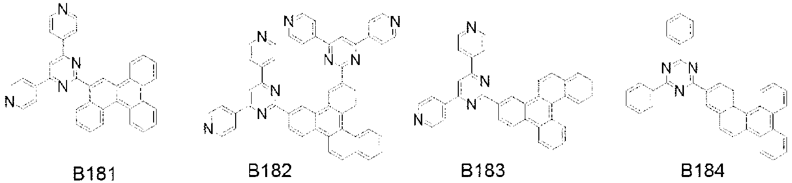

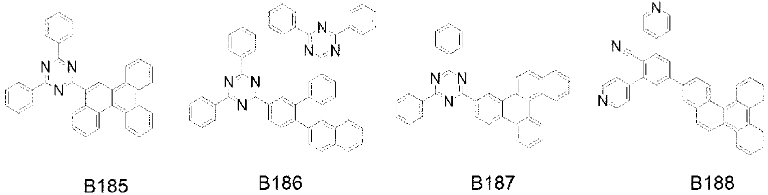

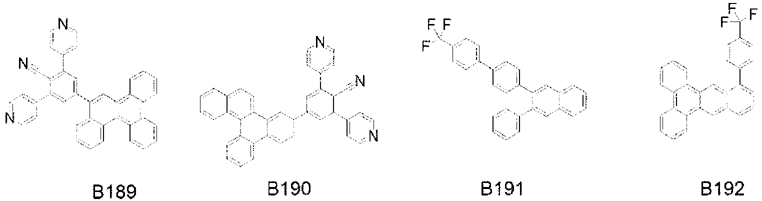

### [B193-B216]

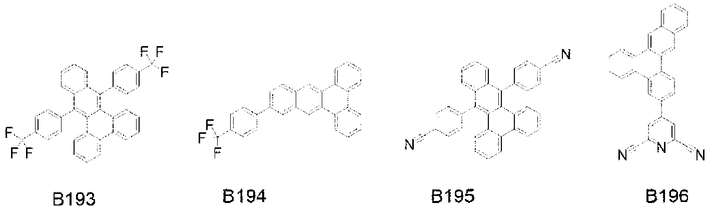

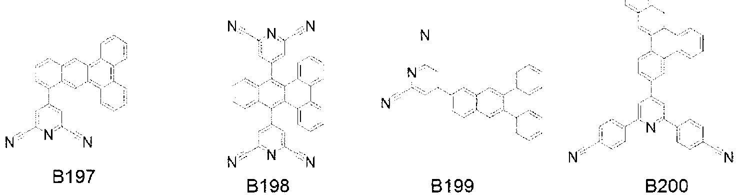

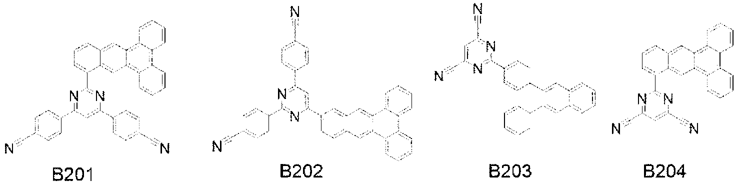

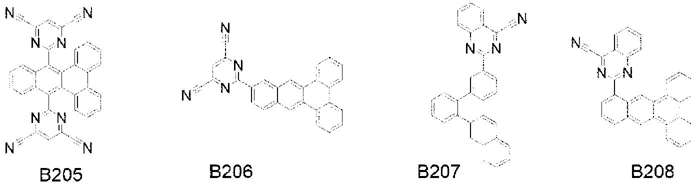

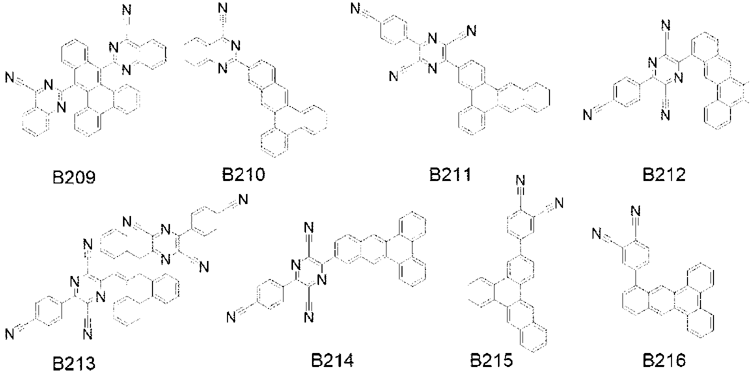

### [B217-B240]

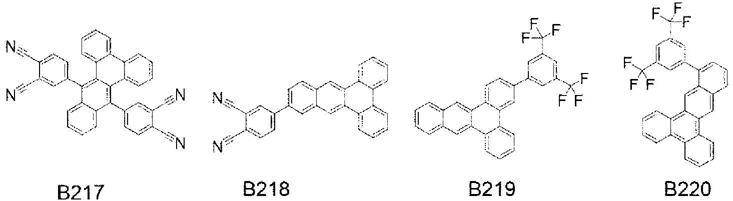

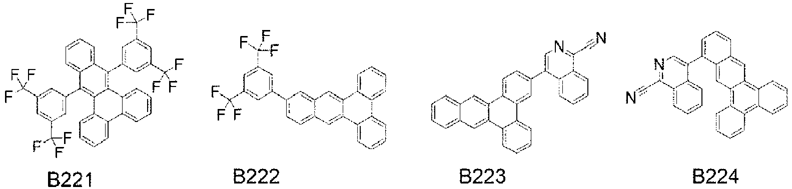

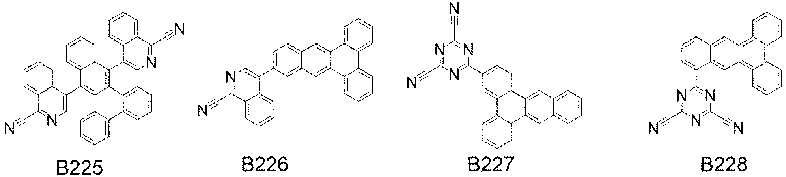

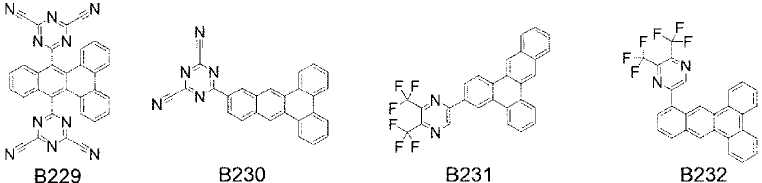

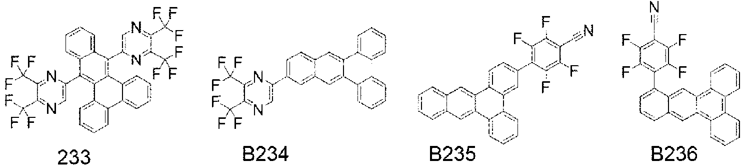

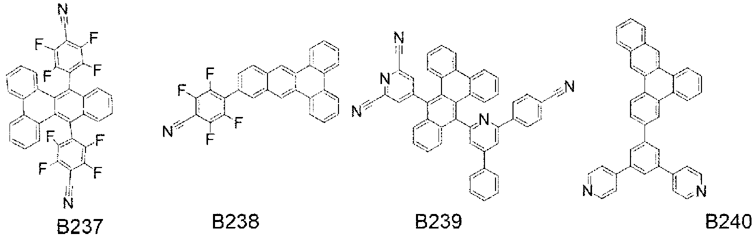

### [B241-B264]

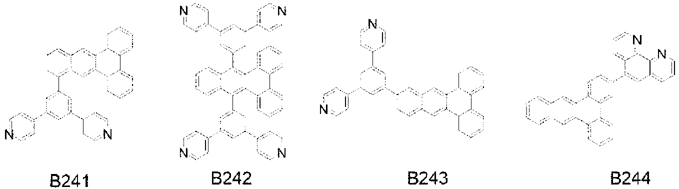

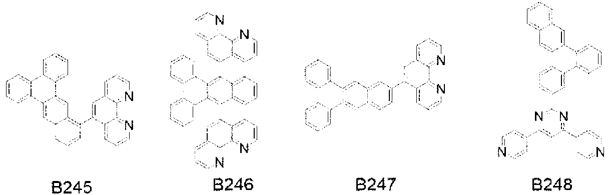

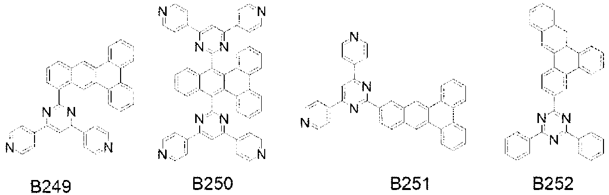

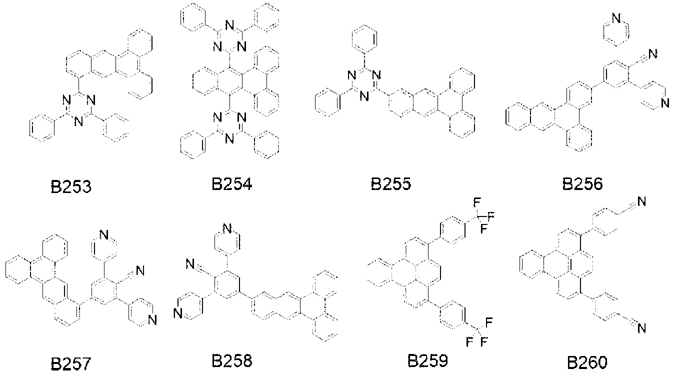

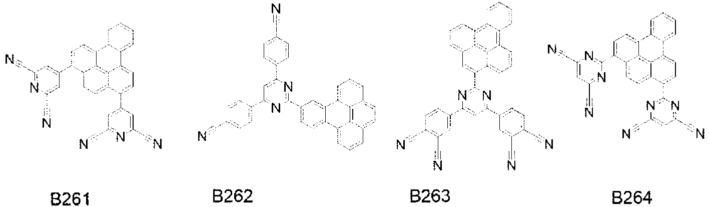

### [B265-B288]

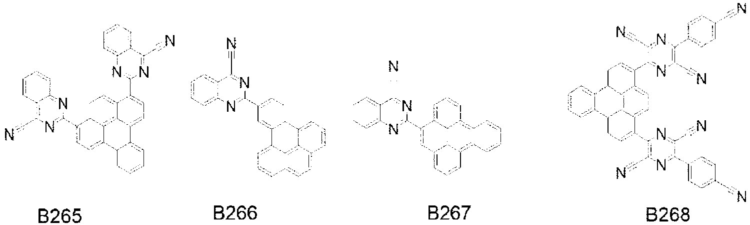

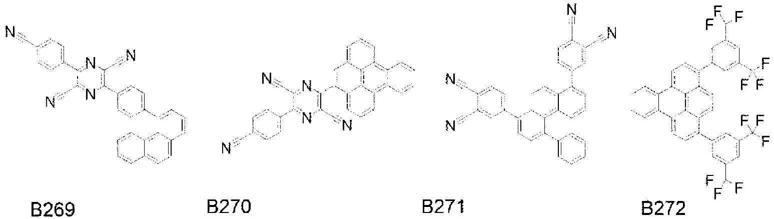

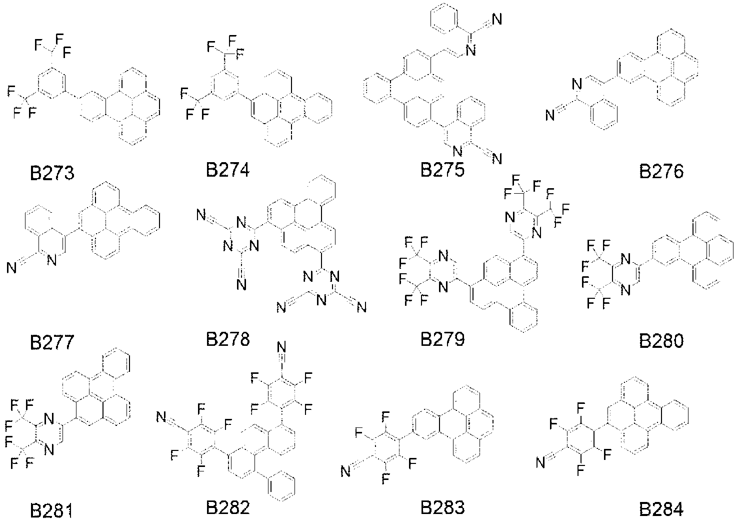

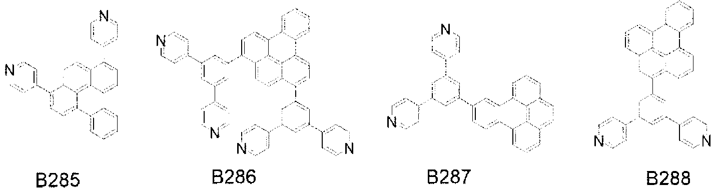

### [B289-B312]

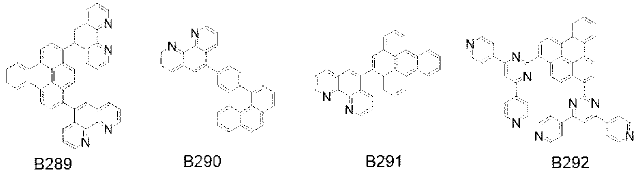

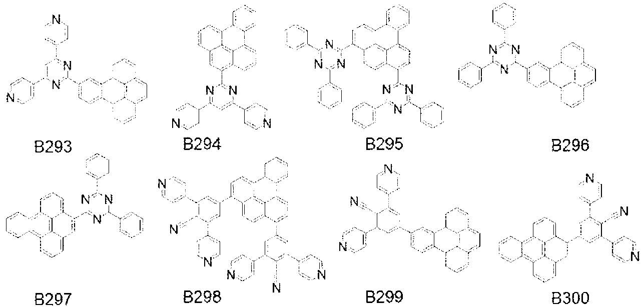

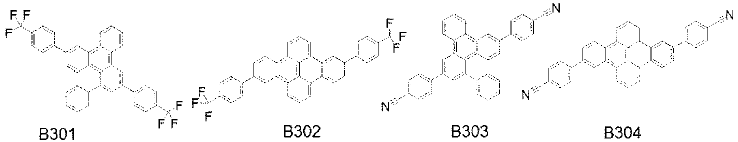

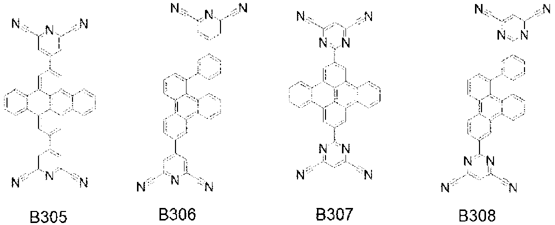

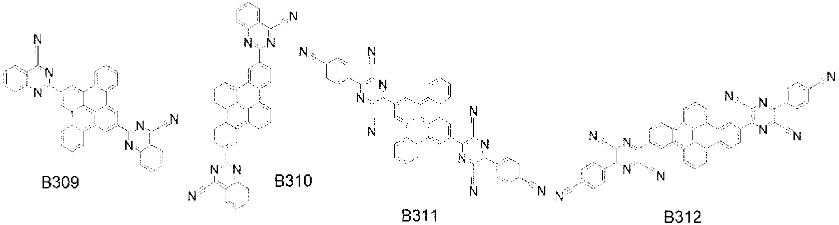

### [B313-B336]

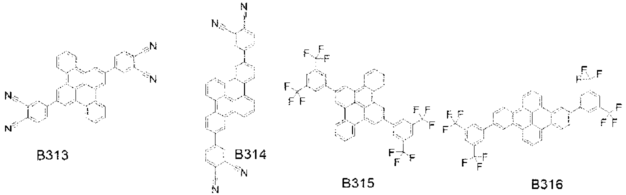

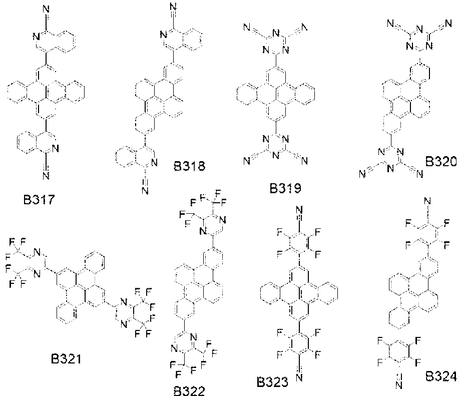

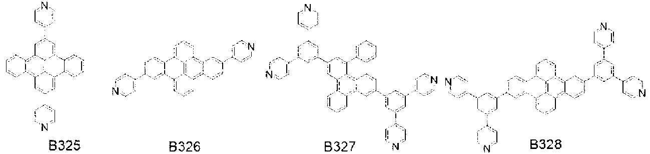

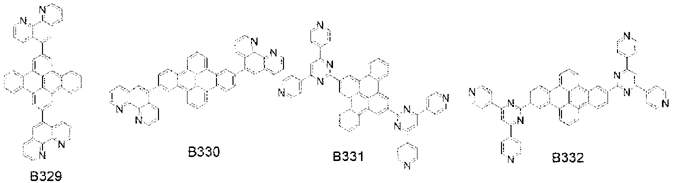

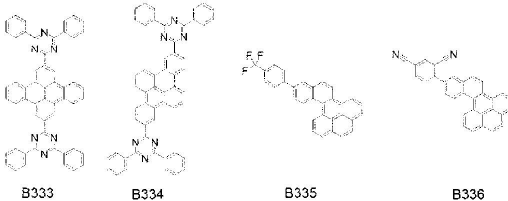

### [B337-B360]

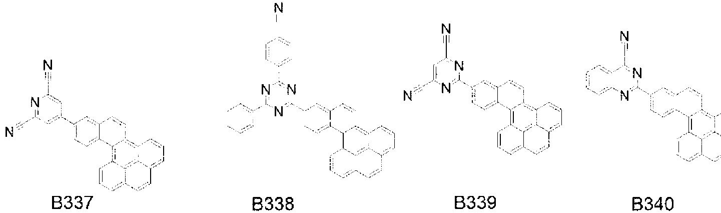

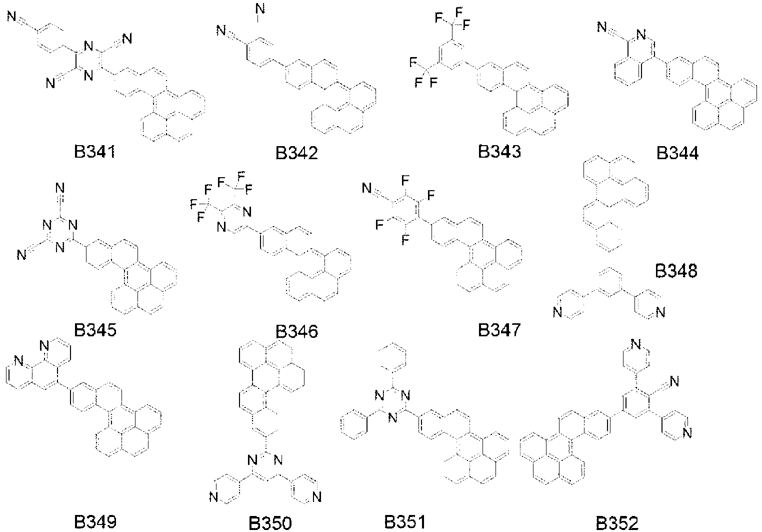

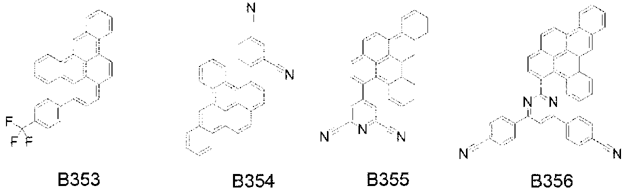

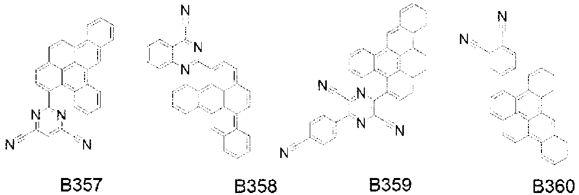

### [B361-B384]

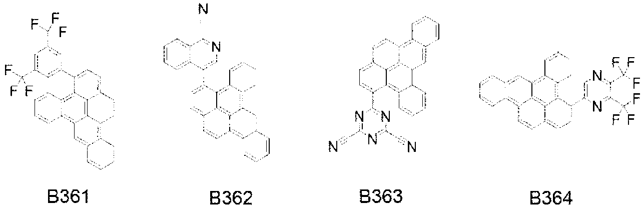

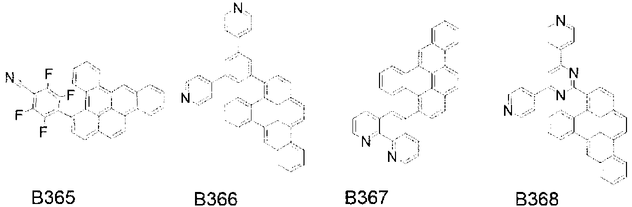

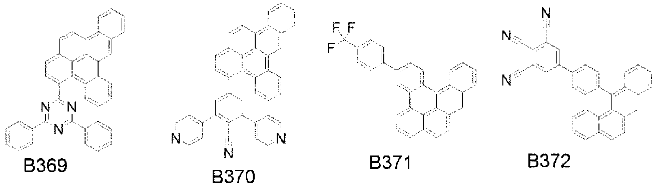

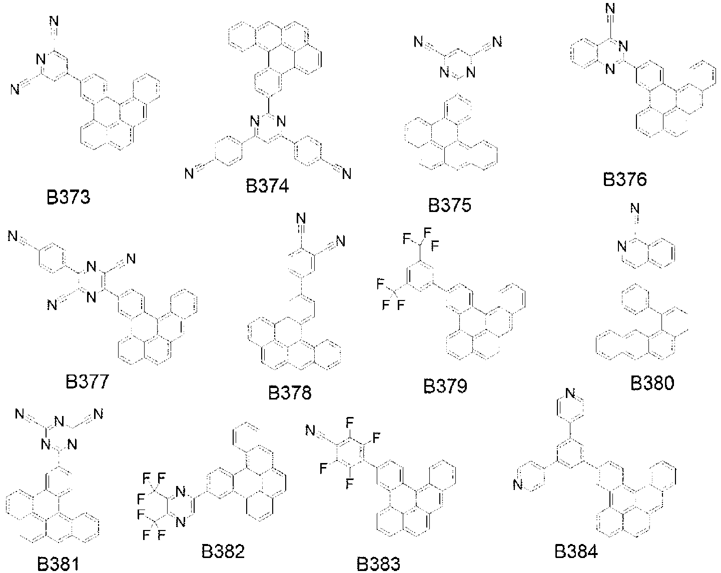

### [B385-B408]

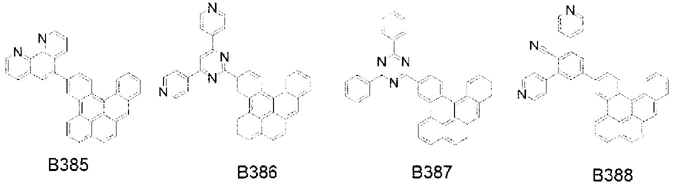

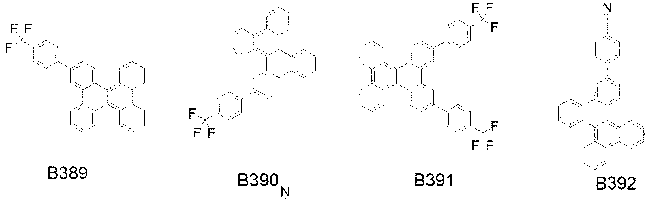

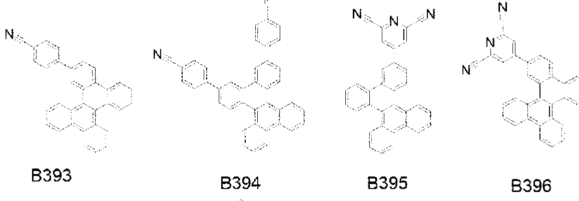

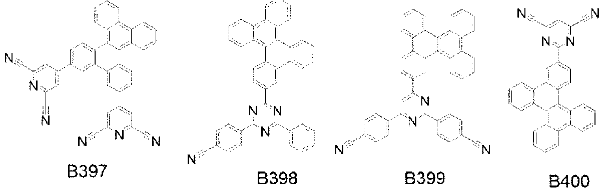

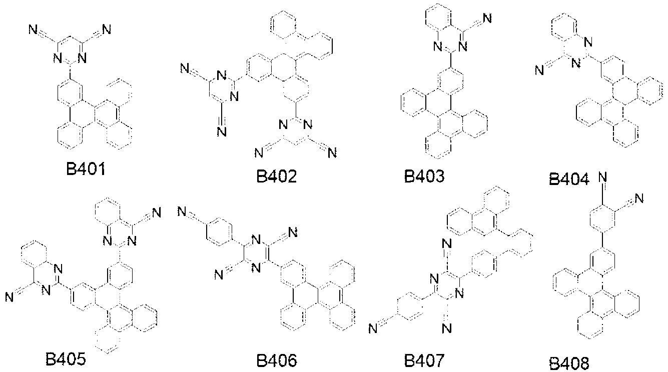

### [B409-B432]

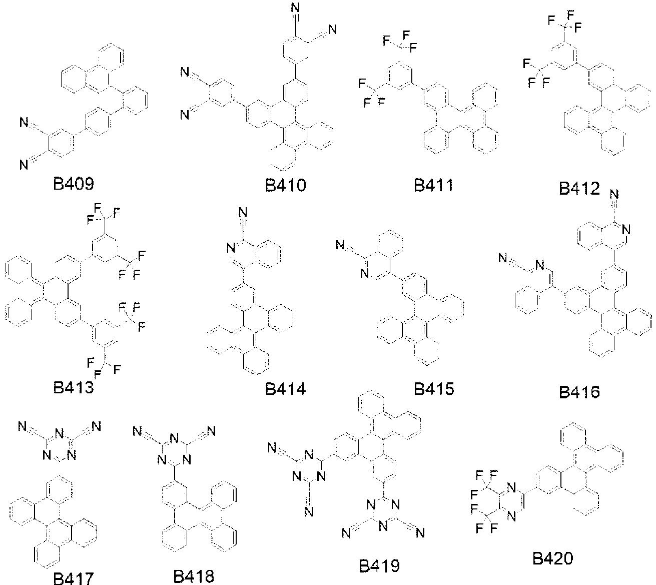

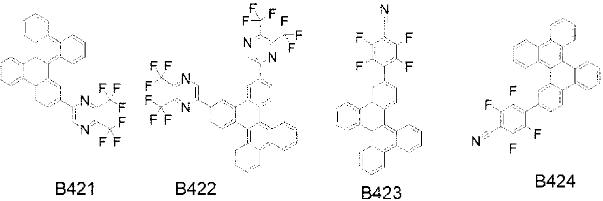

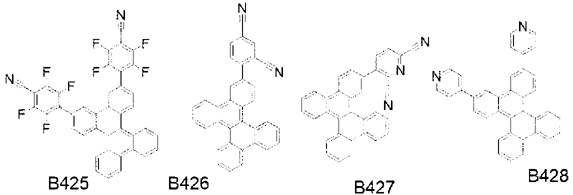

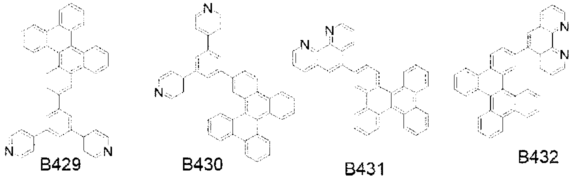

### [B433-B456]

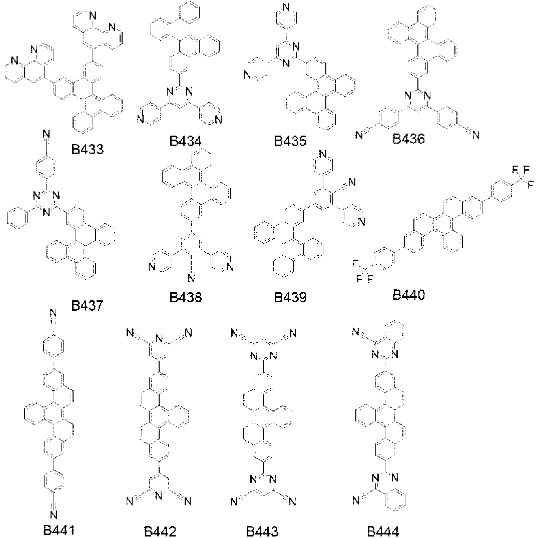

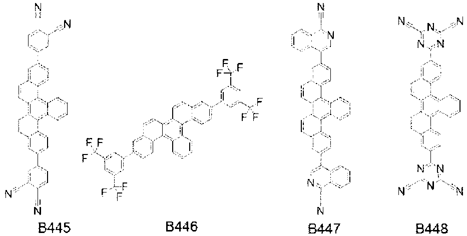

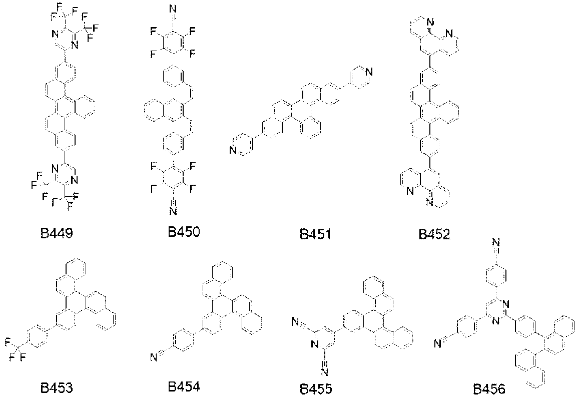

### [B457-B480]

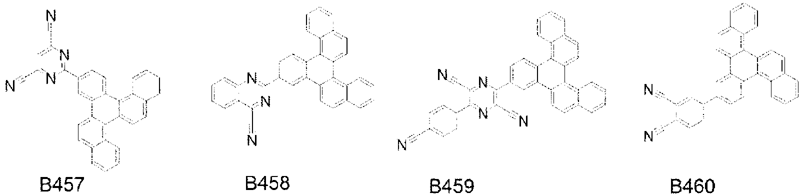

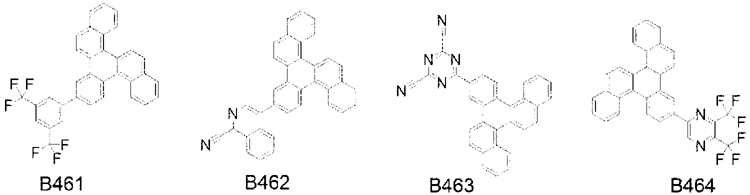

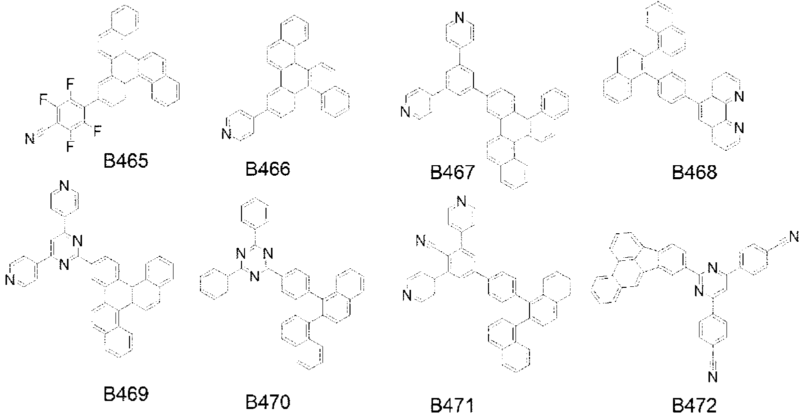

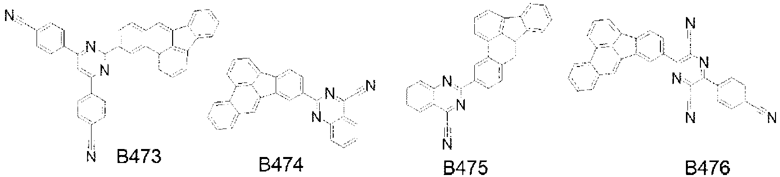

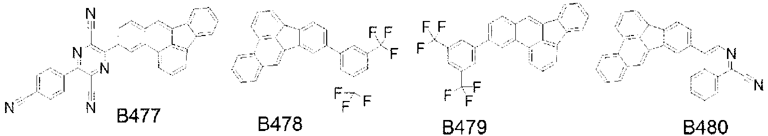

### [B481-B504]

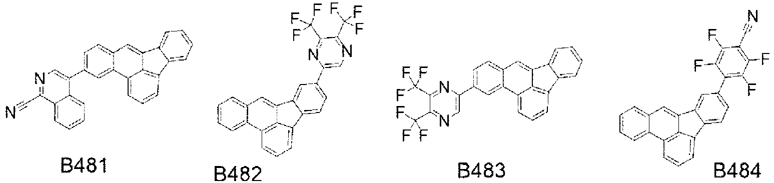

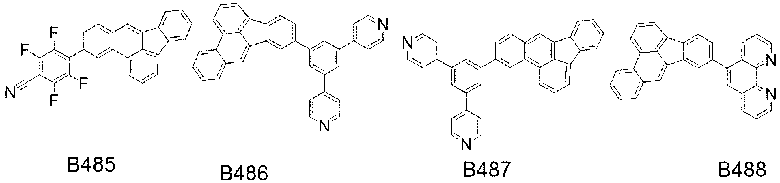

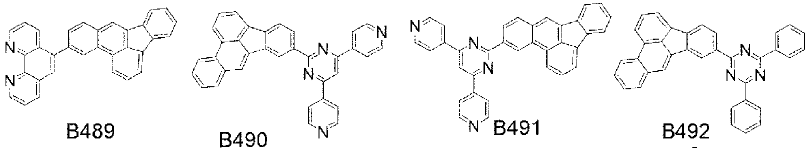

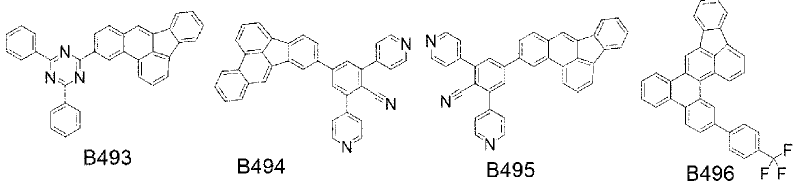

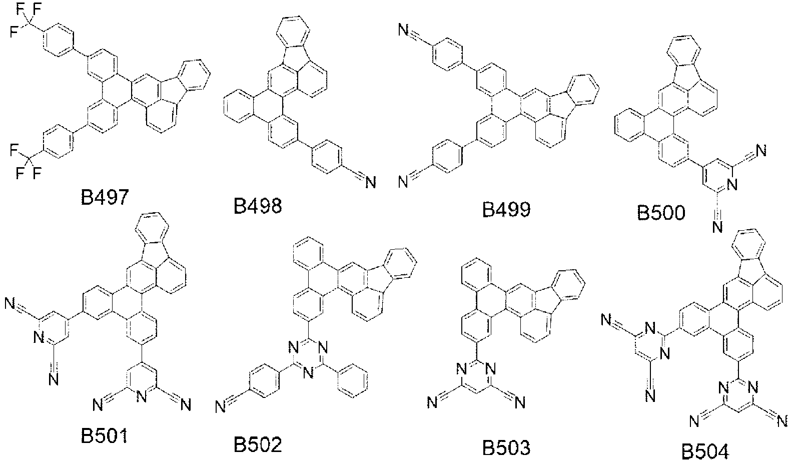

### [B505-B528]

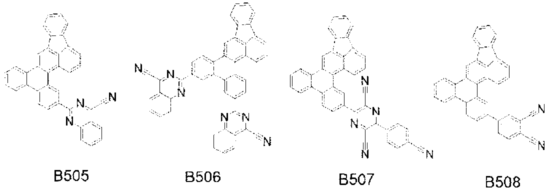

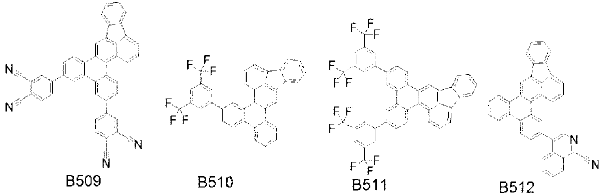

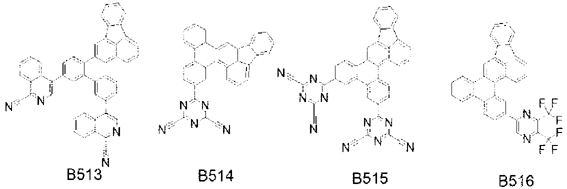

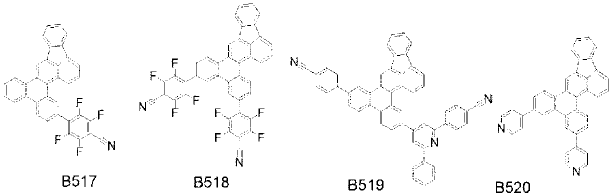

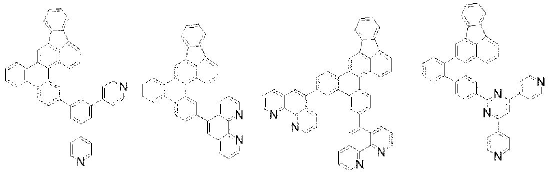

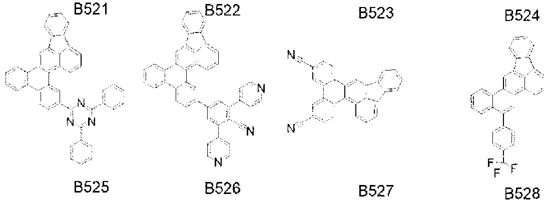

### [B529-B552]

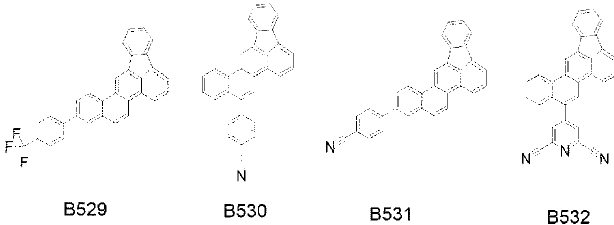

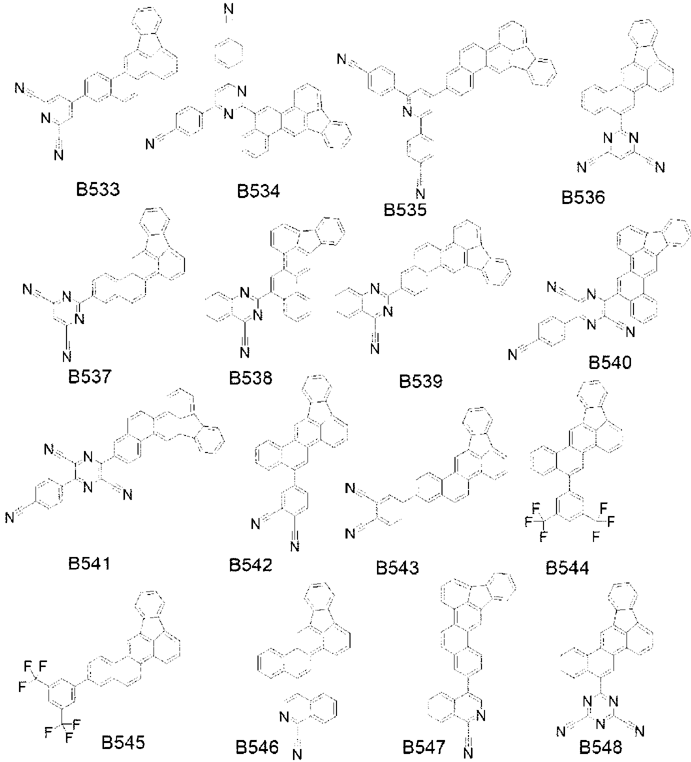

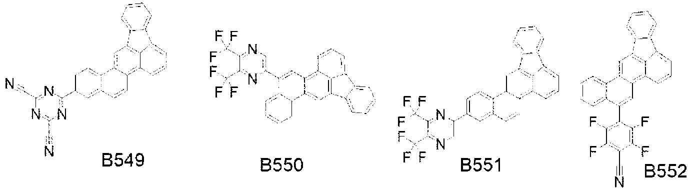

### [B553-B576]

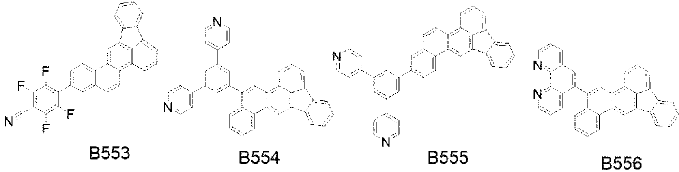

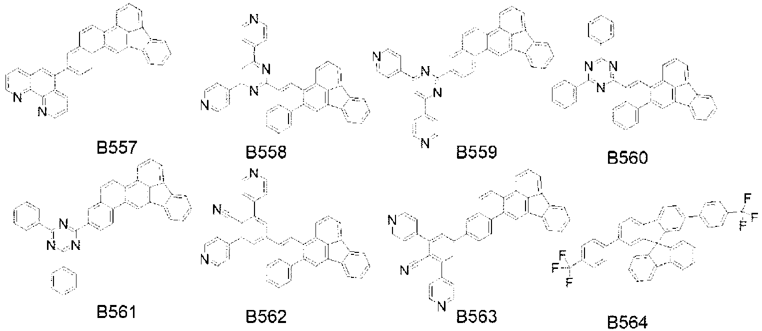

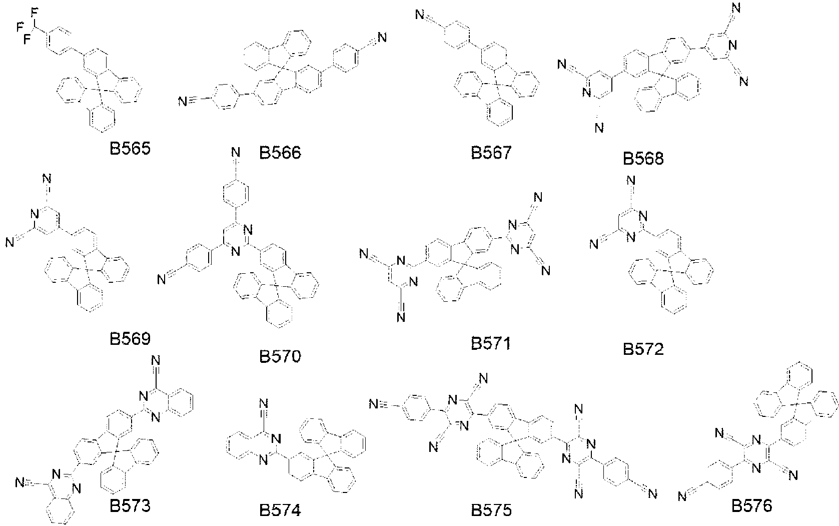

### [B577-B600]

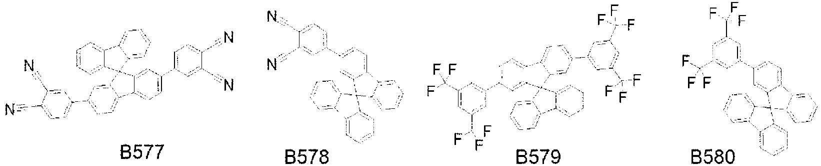

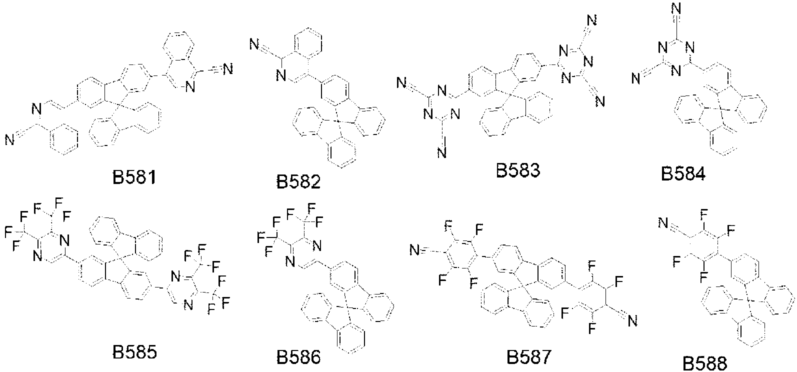

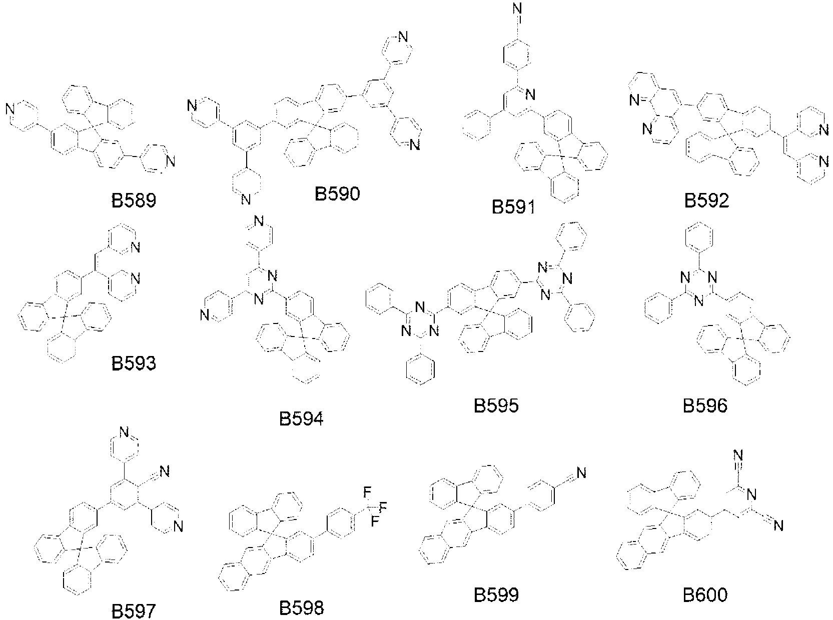

### [B601-B624]

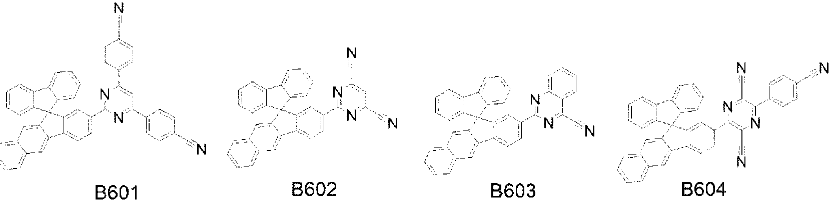

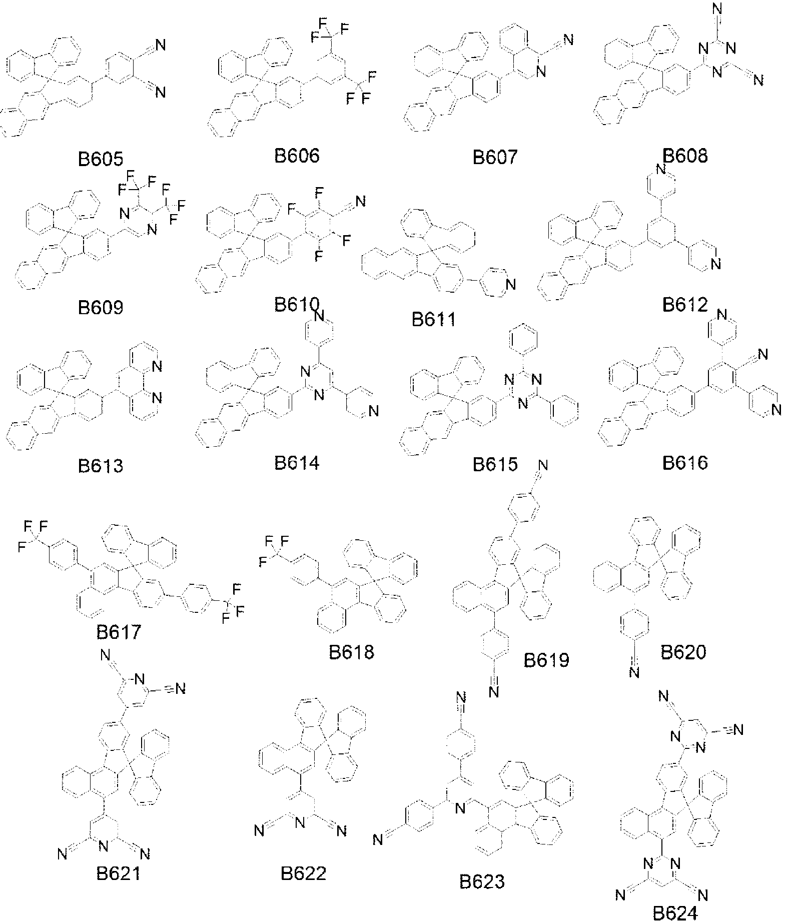

### [B625-B648]

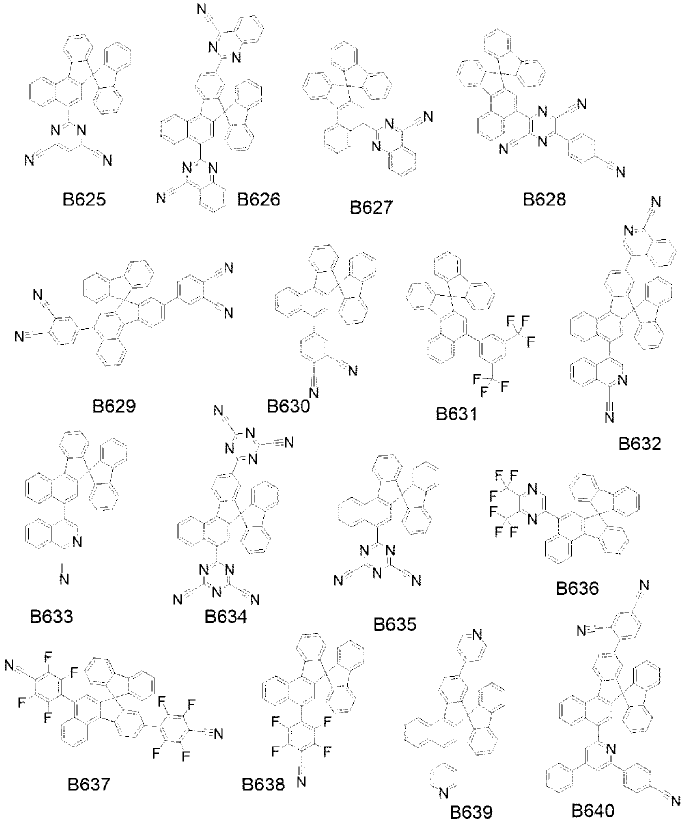

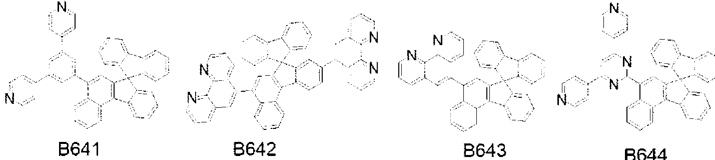

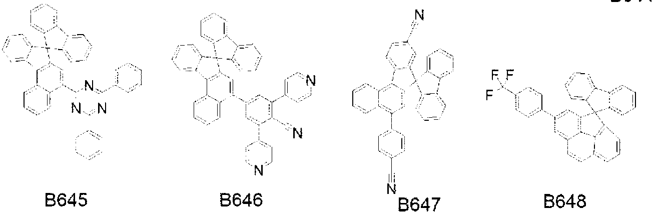

### [B649-B672]

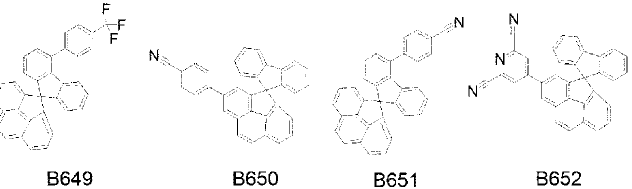

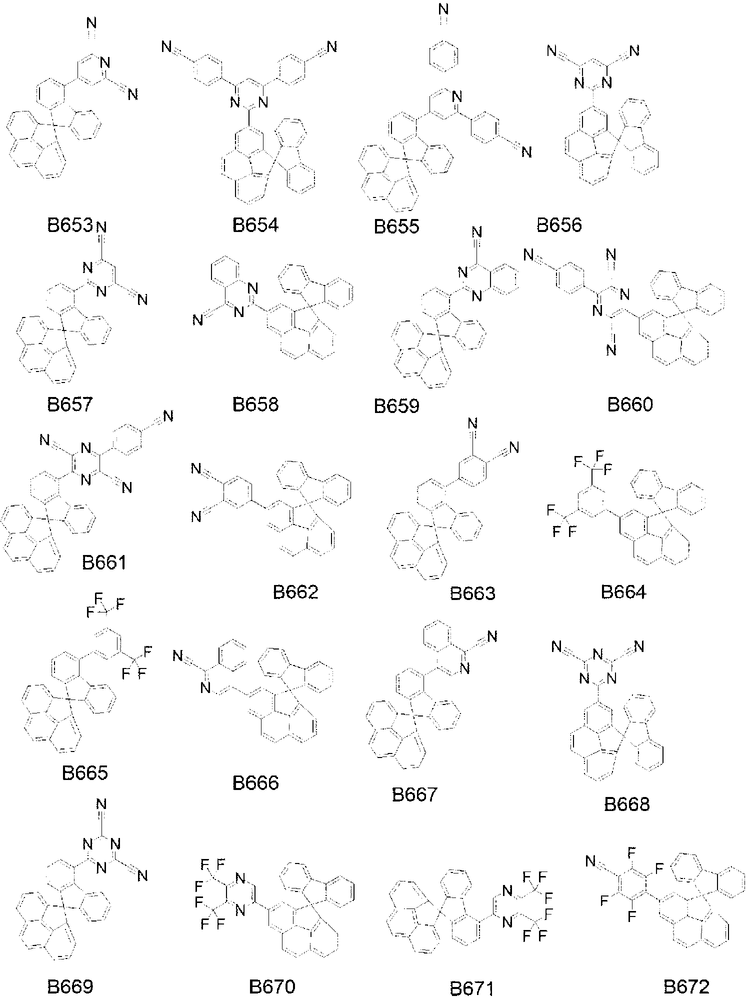

### [B673-B685]

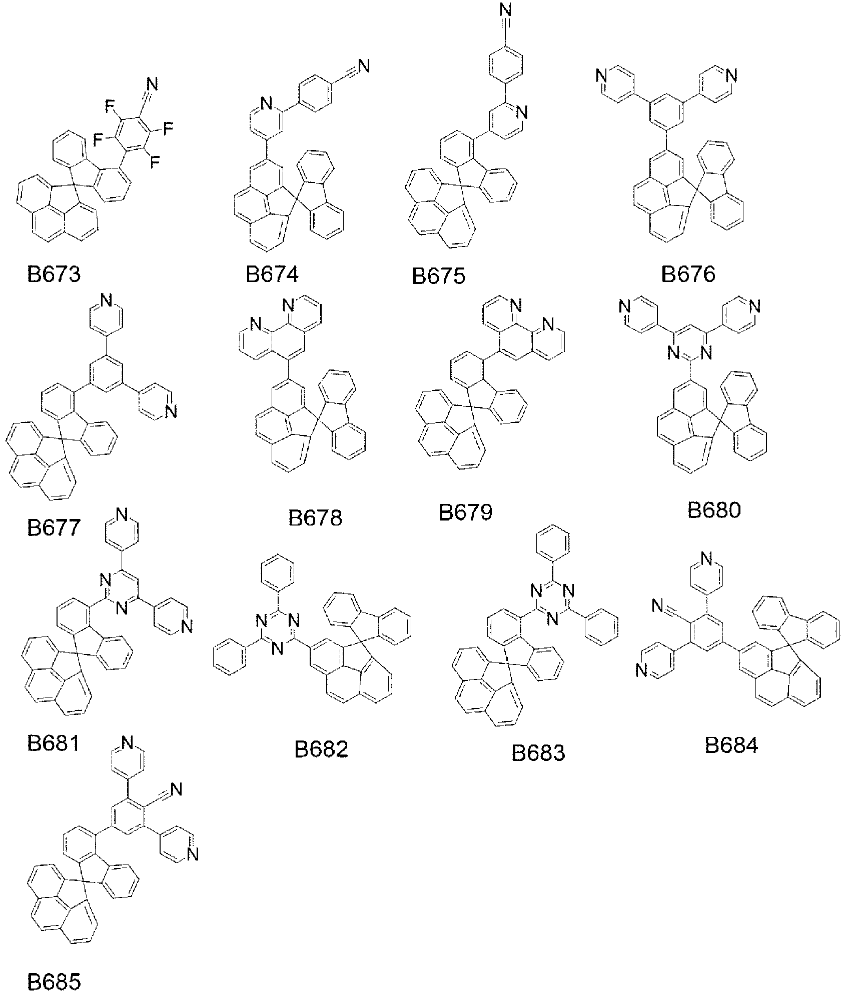

Among the above (B001) through (B685), preferable specific examples of the compound represented by formula (3) include, but not limited to, (B395) through (B397) and (B400) through (B425).

The compounds represented by formula (3) and formula (4-1) can be suitably used, for example, as a photoelectric conversion element material.

### <Photoelectric conversion element material, hole blocking material for photoelectric conversion element>

The following description will discuss the use of the photoelectric conversion element material in accordance with an aspect of the present invention. The photoelectric conversion element material achieves both satisfactory response speed and external quantum efficiency, as described above. Thus, the photoelectric conversion element material can be suitably used for a layer provided in a photoelectric conversion element, suitably used for a photoelectric conversion layer, and suitably used for a hole blocking layer.

The photoelectric conversion element material has a high Tg. This makes it possible to prevent a change in film state such as crystallization due to annealing in production of a photoelectric conversion element. This prevents decreases in external quantum efficiency and dark current in a photoelectric conversion element formed from the photoelectric conversion element material. Therefore, the photoelectric conversion element material can be suitably utilized as a photoelectric conversion element material and a hole blocking material for an imaging element, which are demanded to have resistance to annealing carried out after forming a photoelectric conversion layer.

The photoelectric conversion material can be used, for example, as a photoelectric conversion layer material in an imaging element or a hole blocking material, which is a material for a hole blocking layer in the imaging element.

The photoelectric conversion element material in accordance with an aspect of the present invention includes a skeleton represented by formula (1) or formula (3), or formula (4-1) above. The photoelectric conversion element material and the hole blocking material included in a layer which includes a skeleton represented by formula (1) or (3) or formula (4-1) and which is included in an imaging element contribute to production of a photoelectric conversion element material for an imaging element that is excellent in response speed and external quantum efficiency characteristic.

### <Regarding LUMO level>

In a photoelectric conversion element material, for enhancement in dark current and external quantum efficiency, and improvement in response speed, it may be necessary to rapidly move electric charges generated in a photoelectric conversion layer. For rapid movement of electric charges, it is preferable that a LUMO level of an n-type semiconductor material in the photoelectric conversion layer is close to that of a material used in the hole blocking layer. For example, in a case where fullerene (C60) is used for the photoelectric conversion layer, a LUMO level of the hole blocking layer is preferably -2.2 eV or less and more preferably -2.3 eV or less, as a quantum calculation value obtained by density functional theory (DFT) as described later. The LUMO level of the hole blocking layer is not limited, and only needs to be -5.0 eV or more, and is preferably -4.0 eV or more.

LUMO levels of the photoelectric conversion element materials represented by formula (1) and formula (3) are not particularly limited and, from the viewpoint of suitability for an imaging element, the LUMO level is preferably -2.2 eV or less, more preferably -2.3 eV or less.

In the photoelectric conversion element in accordance with an aspect of the present invention, the LUMO level of the photoelectric conversion element material is a numerical value calculated by quantum chemical calculation. Optimization of the molecular structure and calculation of the LUMO level can be carried out with density functional theory (DFT) under calculation conditions of a B3LYP functional and a 6-31G(d) basis function using a program of Gaussian.

### <Regarding glass transition temperature>

The photoelectric conversion element material used for forming a layer included in the imaging element in accordance with an aspect of the present invention is a photoelectric conversion element material which is represented by formula (1) or formula (3). The glass transition temperature of the photoelectric conversion element materials represented by formulae (1) and (3) is not particularly limited. From the viewpoint of suitability for an imaging element, that is, the photoelectric conversion element for an imaging element, the glass transition temperature is preferably 140°C or higher, more preferably 150°C or higher. The glass transition temperature is a value obtained by differential scanning calorimetry.

A differential scanning calorimeter and test conditions are as follows. Differential scanning calorimeter model: DSC702 available from Hitachi High-Technologies Corporation; Operation conditions: A glass transition temperature is obtained from peaks obtained from two scans at a heating rate of 10°C/min and a temperature range of 40°C to 380°C.

### <Regarding amorphousness>

The photoelectric conversion element material as an imaging element in accordance with an aspect of the present invention or the photoelectric conversion element materials represented by formula (1) and formula (3) each preferably derive a deposition film that forms an amorphous layer. If the deposition film is a crystal layer, the interface with an adjacent layer is not uniform, which causes a defect in the element.

A method for confirming whether or not the deposition film is an amorphous layer is not particularly limited. The confirmation can be made by determination based on the presence or absence of crystallization by visual inspection or by a fact that a sharp diffraction peak is not observed by XRD measurement of the deposition film.

### <Imaging element>

The photoelectric conversion element in accordance with an aspect of the present invention is preferably an imaging element and includes a layer containing the photoelectric conversion element material in accordance with an aspect of the present invention.

A configuration of the imaging element is not particularly limited. Examples thereof include the following configurations (i) through (vi).
(i) First electrode/photoelectric conversion layer/second electrode
(ii) First electrode/hole blocking layer/photoelectric conversion layer/second electrode
(iii) First electrode/photoelectric conversion layer/electron blocking layer/second electrode
(iv) First electrode/hole blocking layer/photoelectric conversion layer/electron blocking layer/second electrode
(v) First electrode/hole blocking layer/photoelectric conversion layer/electron blocking layer/hole transport layer/second electrode
(vi) First electrode/electron transport layer/hole blocking layer/photoelectric conversion layer/electron blocking layer/hole transport layer/second electrode

The following description will discuss, in more detail, the imaging element in accordance with an aspect of the present invention with reference to Fig. 1, using the configuration of the above (v) as an example. Fig. 1 is a schematic cross-sectional view illustrating an example of a laminated structure of an imaging element including a layer containing the photoelectric conversion element material in accordance with an aspect of the present invention.

An imaging element 100 includes a first electrode 1, a hole blocking layer 2, a photoelectric conversion layer 3, an electron blocking layer 4, a hole transport layer 5, and a second electrode 6 in this order. Note, however, that some layers of these layers may be omitted, and conversely, another layer may be added. Among the above layers, the hole blocking layer 2, the photoelectric conversion layer 3, the electron blocking layer 4, and the hole transport layer 5 constitute an organic layer 10.

The imaging element 100 illustrated in Fig. 1 can be specifically an imaging photoelectric conversion element. The imaging element 100 receives, by the photoelectric conversion layer 3 which is a light reception layer, light that enters from below the first electrode 1 which is transparent. The incidence direction of light is not particularly limited. The second electrode 6 may be made transparent, and light may enter through the second electrode 6.

In the imaging element 100, among electric charges (holes and electrons) generated by light reception by the photoelectric conversion layer 3, the electrons move to the first electrode 1 and the holes move to the second electrode 6 by an internal electric field due to a concentration difference of carriers constituting the layers and a difference in work function between the first electrode 1 and the second electrode 6. It is also possible to move electric charges by applying a voltage between the first electrode 1 and the second electrode 6. Thus, the first electrode 1 serves as an electron-collecting electrode, and the second electrode 6 serves as a hole-collecting electrode.

If necessary, each of the layers may be substituted with a layer having another name or function. Examples of the layer having another name or function are as follows. Other names of the hole transport layer can be a hole injection layer, a work function adjustment layer, a hole transport enhancement layer, and the like.

In Fig. 1, a substrate provided on the bottom surface of the first electrode 1 is omitted. The substrate is not particularly limited, and examples thereof include a glass plate, a quartz plate, a plastic plate, and the like. In a case of a configuration in which light enters from the substrate side, the substrate is transparent with respect to the wavelength of light. The substrate may be provided on the second electrode 6 side. The following description will discuss each of the above layers. The following description will discuss each of the above layers.

### [Layer containing photoelectric conversion element material]

An imaging element which is an aspect of the photoelectric conversion element may include the photoelectric conversion element material represented by formula (1) or (3) or formula (4-1) above in at least one layer selected from the group consisting of the photoelectric conversion layer and a layer between the photoelectric conversion layer and the second electrode. In the configuration example illustrated in Fig. 1, the imaging element 100 contains the photoelectric conversion element material in at least one layer selected from the group consisting of the hole blocking layer 2 and the photoelectric conversion layer 3. The imaging element in accordance with an aspect of the present invention preferably contains the photoelectric conversion element material in the hole blocking layer 2. This brings about an effect of rapidly moving necessary electric charges while controlling reverse movement of holes.

The photoelectric conversion element material represented by formula (1) or (3) or formula (4-1) above may be included in a plurality of layers included in the photoelectric conversion element. In a case where an electron transport layer is provided, the electron transport layer may include the photoelectric conversion element material.

The following description will discuss the imaging photoelectric conversion element 100 in which the hole blocking layer 2 contains the photoelectric conversion element material.

### [First electrode 1]

The first electrode 1 is provided on the substrate.

In a case where the imaging element has a configuration in which light passes through the first electrode and enters the photoelectric conversion layer 3, the first electrode 1 may be formed by a transparent material that allows light to pass therethrough or substantially allows light to pass therethrough.

The transparent material used for a lower electrode, which is the first electrode 1, is not particularly limited. Examples thereof include indium tin oxide (ITO), indium zinc oxide (IZO), tin oxide, aluminum-doped tin oxide, magnesium indium oxide, nickel tungsten oxide, other metal oxides, metal nitrides such as gallium nitride, metal selenides such as zinc selenide, and metal sulfides such as zinc sulfide.

Note that, in a case where the imaging element 100 has a configuration in which light enters the photoelectric conversion layer 3 from only the second electrode 6 side, a light transmission characteristic of the first electrode 1 is not important. Therefore, examples of materials used in the first electrode 1 in this case includes gold, iridium, molybdenum, palladium, platinum, and the like.

### [Hole blocking layer 2]

The hole blocking layer 2 is provided between the first electrode 1 and the photoelectric conversion layer 3 which is a light reception layer described later.

The hole blocking layer 2 has a role of transporting an electron generated in the photoelectric conversion layer 3 to the first electrode 1 and a role of blocking movement of a hole from the photoelectric conversion layer 3 to the first electrode 1 to which an electron is to be transported. The hole blocking layer 2 may also have a role of blocking hole injection from the first electrode 1 in some applications.

The hole blocking layer 2 may include, in addition to the photoelectric conversion element material represented by the foregoing formula (1) and formula (3) or formula (4-1), a conventionally known hole blocking material (electron transport material). Examples of the conventionally known hole blocking material (electron transport material) include bis(8-hydroxyquinolinate)manganese, tris(8-hydroxyquinolinate)aluminum, tris(2-methyl-8-hydroxyquinolinate)aluminum, BCP(2,9-dimethyl-4,7-diphenyl-1,10-phenanthroline), Bphen(4,7-diphenyl-1,10-phenanthroline), BAlq(bis(2-methyl-8-quinolinolate)-4-(phenylphenolate)aluminum), 4,6-bis(3,5-di(pyridin-4-yl)phenyl)-2-methylpyrimidine, N,N'-diphenyl-1,4,5,8-naphthalenetetracarboxylic diimide, and N,N'-di(4-pyridyl)-1,4,5,8-naphthalenetetracarboxylic diimide, and the like.

The hole blocking layer 2 may have a single layer structure constituted by one material or two or more materials, or may have a laminated structure constituted by a plurality of layers of the same composition or different compositions.

### [Photoelectric conversion layer 3]

The photoelectric conversion layer 3 which is a light reception layer is provided between the hole blocking layer 2 and the electron blocking layer 4 described later. A material of the photoelectric conversion layer 3 can be a material having a photoelectric conversion function.

The photoelectric conversion layer 3 may have a single layer structure constituted by one material or two or more materials, or may have a laminated structure constituted by a plurality of layers of the same composition or different compositions. Among those, in order to enhance the photoelectric conversion efficiency, it is preferable that the photoelectric conversion layer 3 is constituted by a layer containing at least two types of materials (organic components).

Examples of a material used for the photoelectric conversion layer 3 having a single layer structure constituted by one type of material include (i) coumarin and a derivative thereof, quinacridone and a derivative thereof, and phthalocyanine and a derivative thereof.

Examples of materials used for the photoelectric conversion layer 3 having a single layer structure constituted by two types of materials include a combination of the above described (i) coumarin and a derivative thereof, quinacridone and a derivative thereof, and phthalocyanine and a derivative thereof and (ii) fullerene and a derivative thereof. The photoelectric conversion layer 3 constituted by these materials may be formed by deposition in a state in which powder is mixed in advance, or may be formed by codeposition at an arbitrary proportion.

Examples of materials used for the photoelectric conversion layer 3 having a single layer structure constituted by three types of materials include a combination of the above described (i) coumarin and a derivative thereof, quinacridone and a derivative thereof, and phthalocyanine and a derivative thereof, (ii) fullerene and a derivative thereof, and (iii) a hole transport material. The photoelectric conversion layer 3 constituted by these materials may be formed by deposition in a state in which powder is mixed in advance, or may be formed by codeposition at an arbitrary proportion.

Specific examples of (i) the coumarin derivative include coumarin 6 and coumarin 30. Specific examples of the quinacridone derivative include N,N-dimethylquinacridone. Specific examples of the phthalocyanine derivative include boron subphthalocyanine chloride and boron subnaphthalocyanine chloride (SubNC).

Specific examples of (ii) the fullerene and a derivative thereof include [60]fullerene, [70]fullerene, and methyl [6,6]-phenyl-C61-butyrate ([60]PCBM).

The (iii) hole transport material may be a known hole transport material. Examples of the hole transport material include an aromatic tertiary amine compound, a naphthalene compound, an anthracene compound, a tetracene compound, a pentacene compound, a phenanthrene compound, a pyrene compound, a perylene compound, a fluorene compound, a carbazole compound, an indole compound, a pyrrole compound, a picene compound, a thiophene compound, a benzotrifuran compound, a benzotrithiophene compound, a naphthodithiophene compound, a naphthothienothiophene compound, a benzodifuran compound, a benzodithiophene compound, a benzothiophene compound, a naphthobisbenzothiophene compound, a chrysenodithiophene compound, a benzothienobenzothiophene compound, an indolocarbazole compound, and the like. Among these, the fluorene compound, the naphthodithiophene compound, the naphthothienothiophene compound, the benzodifuran compound, the benzothiophene compound, the naphthobisbenzothiophene compound, the chrysenodithiophene compound, the benzothienobenzothiophene compound, the indolocarbazole compound, and the like are preferable, the fluorene compound, the chrysenodithiophene compound, the benzothienobenzothiophene compound, and the indolocarbazole compound are more preferable.

Specific examples of the hole transport material include 9,9'-(9,9'-spirobi[9H-fluorene]-2,7'-diyl)bis[9H-carbazole], 2,7-diphenyl[1]benzothieno[3,2-b][1]benzothiophene (DiPh-BTBT), a benzo[1,2-b:3,4-b':5,6-b"]trifuran compound, a benzo[1,2-b:3,4-b':5,6-b"]trithiophene compound, naphtho[1,2-b:5,6-b']dithiophene, naphtho[2,3-b]naphtho[2',3':4,5]thieno[2,3-d]thiophene, benzo[1,2-b:4,5-b']difuran, benzo[1,2-b:4,5-b']dithiophene, benzo[1,2-b:4,5-b']bis[1]benzothiophene, naphtho[1,2-b:5,6-b']bis[1]benzothiophene, chryseno[1,2-b:8,7-b']dithiophene, [1]benzothieno[3,2-b][1]benzothiophene, the following compounds (ic-1), (ic-2), and (ic-3), and the like.

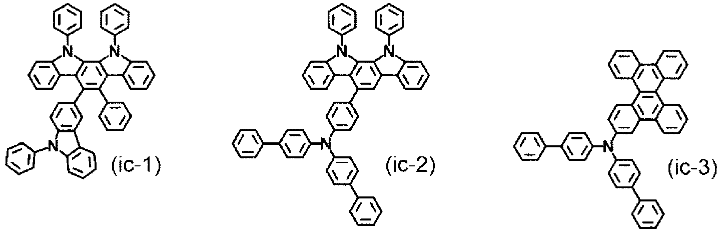

The photoelectric conversion element material is not limited to be contained only in the photoelectric conversion layer. For example, the photoelectric conversion element material may be contained in a layer (the hole blocking layer 2 or the electron blocking layer 4) adjacent to the photoelectric conversion layer 3.

### [Electron blocking layer 4]

The electron blocking layer 4 is provided between the photoelectric conversion layer 3 and the hole transport layer 5.

The electron blocking layer 4 has a role of transporting a hole generated in the photoelectric conversion layer 3 from the photoelectric conversion layer 3 to the second electrode 6 and a role of blocking movement of an electron generated in the photoelectric conversion layer 3 to the second electrode 6 side. The electron blocking layer 4 may also have a role of blocking electron injection from the second electrode 6 in some applications.

The electron blocking layer 4 may have a single layer structure constituted by one material or two or more materials, and may have a laminated structure constituted by a plurality of layers of the same composition or different compositions. For example, the electron blocking layer 4 may have a two-layer structure including: a layer which is adjacent to the photoelectric conversion layer 3 and is composed of a material specialized in electron blocking property; and a layer which is adjacent to the hole transport layer 5 and is composed of a material specialized in hole transport property.

The electron blocking layer 4 preferably contains a known hole transport material. Examples of the known hole transport material include materials identical with those used in the photoelectric conversion layer 3 described above.

### [Hole transport layer 5]

The hole transport layer 5 is provided between the electron blocking layer 4 and the second electrode 6 described later. The hole transport layer 5 is provided to facilitate hole transport from the electron blocking layer 4 to the second electrode 6. Facilitation of hole transport is achieved when a hole transport material changes an internal electric field by interaction with a surrounding material. In a case where the second electrode 6 is formed by a sputtering method, the hole transport layer 5 has a role of reducing damage to the organic layer (e.g., the electron blocking layer 4) during sputtering.

The hole transport layer 5 may be a known material. Examples thereof include naphthalene-1,4,5,8-tetracarboxylic dianhydride (NTCDA), 2,3,6,7,10,11-hexacyano-1,4,5,8,9,12-hexaazatriphenylene (HATCN), and the like.

The hole transport layer 5 may have a single layer structure constituted by one material or two or more materials. For example, the hole transport layer 5 may contain a conventionally known hole transport material and the above described material. Examples of the conventionally known hole transport material include materials identical with those used in the photoelectric conversion layer 3 described above.

### [Second electrode 6]

The second electrode 6 is provided on the electron blocking layer 4.

Examples of a material for the second electrode 6 include sodium, a sodium-potassium alloy, magnesium, lithium, a magnesium/copper mixture, a magnesium/silver mixture, a magnesium/aluminum mixture, a magnesium /indium mixture, an aluminum/aluminum oxide (Al₂O₃) mixture, indium, a lithium/aluminum mixture, gold, rare earth metal, and the like.

### [Method for forming each layer]

Except for the first electrode 1 and the second electrode 6 described above, each of the layers can be formed by making a material of that layer (if necessary, with a material such as a binding resin and a solvent) into a thin film by a known method such as, for example, a vacuum deposition method, a spin coating method, a casting method, and a Langmuir-Blodgett (LB) method.

There is no particular limitation on the film thickness of each of the layers thus formed, and the film thickness can be selected as appropriate depending on the situation. The film thickness is usually in a range of 5 nm or more and 5 µm or less.

The first electrode 1 which is a lower electrode and the second electrode 6 which is an upper electrode can be formed by making an electrode material into a thin film by a method such as deposition or sputtering. During deposition or sputtering, a pattern may be formed via a mask of an intended shape. After forming a thin film by deposition, sputtering, or the like, a pattern of an intended shape may be formed by photolithography.

A film thickness of the first electrode 1 and the second electrode 6 is preferably 1 µm or less, more preferably 10 nm or more and 200 nm or less.

The imaging element including the photoelectric conversion element in accordance with an aspect of the present invention can be applied, for example, to imaging elements of a digital camera and a digital video camera, and to an imaging element built in a mobile phone or the like.

Aspects of the present invention can also be expressed as follows:
A photoelectric conversion element in accordance with aspect 1 of the present invention is a photoelectric conversion element including a layer containing a photoelectric conversion element material,
the photoelectric conversion element material being represented by formula (1) below: 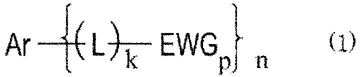
where
EWG represents an electron-withdrawing group,
Ar represents a condensed ring aromatic hydrocarbon group having 16 to 40 carbon atoms,
L represents an aromatic hydrocarbon group having 6 to 30 carbon atoms,
n represents an integer of 1 to 8,
k represents an integer of 0 to 2,
p represents an integer of 1 to 8,
in a case where k is 0, p is 1,
in a case where there are two or more EWG, the two or more EWG are identical or different electron-withdrawing groups, and
each of Ar, L, and EWG has at least one substituent or has no substituent.

In the photoelectric conversion element in accordance with aspect 2 of the present invention, it is preferable in the aspect 1 that: the EWG is selected from a cyano group, a fluorine atom, a fluoroalkyl group, and a heteroaryl group that has 3 to 30 carbon atoms and that includes a nitrogen atom.

In the photoelectric conversion element in accordance with aspect 3 of the present invention, it is preferable in the aspect 1 or 2 that: the heteroaryl group that has 3 to 30 carbon atoms and includes a nitrogen atom is substituted by a group selected from a cyano group, a fluorine atom, and a fluoroalkyl group.

In the photoelectric conversion element in accordance with aspect 4 of the present invention, it is preferable in one of the aspects 1 through 3 that: k is an integer of 0 or 1.

In the photoelectric conversion element in accordance with aspect 5 of the present invention, it is preferable in one of the aspects 1 through 4 that: k is 1; and the EWG is at least one group selected from a cyano group, a fluorine group, and a fluoroalkyl group.

In the photoelectric conversion element in accordance with aspect 6 of the present invention, it is preferable in one of the aspects 1 through 5 that: Ar is a condensed ring aromatic hydrocarbon group which encompasses four or more 6-membered rings.

In the photoelectric conversion element in accordance with aspect 7 of the present invention, it is preferable in one of the aspects 1 through 6 that: Ar is selected from (i) a condensed ring aromatic hydrocarbon group which is selected from a triphenylene group, a fluoranthene group, and a spirofluoren group and (ii) a condensed ring aromatic hydrocarbon group which encompasses a triphenylene group, a fluoranthene group, and a spirofluoren group.

In the photoelectric conversion element in accordance with aspect 8 of the present invention, it is preferable in one of the aspects 1 through 7 that: Ar represents a condensed ring aromatic hydrocarbon group having 20 to 40 carbon atoms, the condensed ring aromatic hydrocarbon group having the at least one substituent or having no substituent.

In the photoelectric conversion element in accordance with aspect 9 of the present invention, it is preferable in one of the aspects 1 through 8 that Ar is represented by formulae (2-1), (2-2), and (2-3) below; 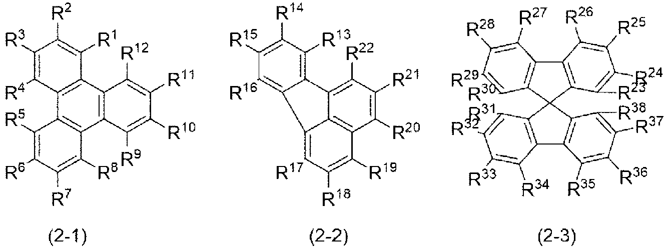 where
R¹ through R³⁸ are each independently selected from a hydrogen atom, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R¹ through R³⁸ form a ring by bonding of two adjacent groups thereof, and
R¹ through R³⁵ each independently has at least one substituent or has no substituent.

In the photoelectric conversion element in accordance with aspect 10 of the present invention, it is preferable in one of the aspects 1 through 9 that: the photoelectric conversion element material has a LUMO level of -2.2 eV or less, the LUMO level being a quantum calculation value which is calculated by density functional theory (DFT) using a B3LYP functional and a 6-31G(d) basis function.

In the photoelectric conversion element in accordance with aspect 11 of the present invention, it is preferable in one of the aspects 1 through 10 that: a glass transition temperature of the photoelectric conversion element material is 140°C or higher.

In the photoelectric conversion element in accordance with aspect 12 of the present invention, it is preferable in one of the aspects 1 through 11 that: the layer containing the photoelectric conversion element material is a hole blocking layer.

The photoelectric conversion element in accordance with one of the aspects 1 through 13 of the present invention is preferably an imaging element.

The photoelectric conversion element material in accordance with aspect 14 of the present invention can be a photoelectric conversion element material for forming the layer included in the photoelectric conversion element in accordance with one of the aspects 1 through 13, the photoelectric conversion element material being represented by the formula (1).

The photoelectric conversion element material in accordance with aspect 15 of the present invention can be a photoelectric conversion element material for an imaging element.

In the photoelectric conversion element material in accordance with aspect 16 of the present invention, the photoelectric conversion element material in accordance with the aspect 14 can be a hole blocking material.

The photoelectric conversion element in accordance with aspect 17 of the present invention preferably includes a fullerene-containing layer which is disposed between an electrode and the hole blocking layer that are included in the photoelectric conversion element in accordance with the aspect 12.

A photoelectric conversion element in accordance with aspect 18 of the present invention is a photoelectric conversion element including a layer containing a photoelectric conversion element material, in which
the photoelectric conversion element material is a compound represented by formula (3) below: 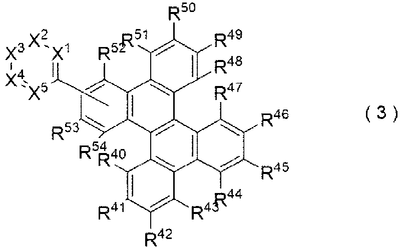
where
X¹ through X⁵ each represent a nitrogen atom or CR⁵⁵,
the number of nitrogen atoms in X¹ through X⁵ is an integer of 0 to 3,
R⁴⁰ through R⁵⁵ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R⁴⁰ through R⁵⁵ form a ring by bonding of two adjacent groups thereof,
at least one R⁵⁵ is selected from a cyano group, a halogen atom, and an alkyl halide group,
in a case where X¹ through X⁵ are all CR⁵⁵, two or more of R⁵⁵ are selected from a cyano group, a halogen atom, and an alkyl halide group, and
R⁴⁰ through R⁵⁵ each independently have further at least one substituent or have no substituent.

In the compound in accordance with aspect 19 of the present invention, it is preferable in the aspect 18 that: each R⁵⁵ is independently selected from a hydrogen atom, a cyano group, a halogen atom, an alkyl halide group, an alkyl group having 1 to 20 carbon atoms, and a cycloalkyl group having 3 to 20 carbon atoms.

In the compound in accordance with aspect 20 of the present invention, it is preferable in the aspect 18 or 19 that: one of R⁵⁵ is a cyano group.

In the compound in accordance with aspect 21 of the present invention, it is preferable in one of the aspects 18 through 20 that:
the halogen atom is a fluorine atom; and
one of the aromatic hydrocarbon group having 6 to 30 carbon atoms and the heteroaryl group having 3 to 30 carbon atoms is substituted or not substituted by a group selected from a cyano group, a fluorine group, and a fluoroalkyl group.

In the compound in accordance with aspect 22 of the present invention, it is preferable in one of the aspects 18 through 21 that: the photoelectric conversion element material has a LUMO level of -2.2 eV or less, the LUMO level being a quantum calculation value which is calculated by density functional theory (DFT) using a B3LYP functional and a 6-31G(d) basis function.

In the compound in accordance with aspect 23 of the present invention, it is preferable in one of the aspects 18 through 22 that: a glass transition temperature of the photoelectric conversion element material is 140°C or higher.

The photoelectric conversion element material in accordance with aspect 24 of the present invention preferably contains the compound in accordance with one of the aspects 18 through 23.

The photoelectric conversion element material in accordance with aspect 25 of the present invention is, in the aspect 24, preferably a hole blocking material.

The photoelectric conversion element material in accordance with aspect 26 of the present invention preferably includes a layer containing the photoelectric conversion element material in accordance with the aspect 24 or 25.

The photoelectric conversion element in accordance with aspect 27 of the present invention can be, in the aspect 26, an imaging element.

The compound in accordance with aspect 28 of the present invention is represented by formula (4-1) below: 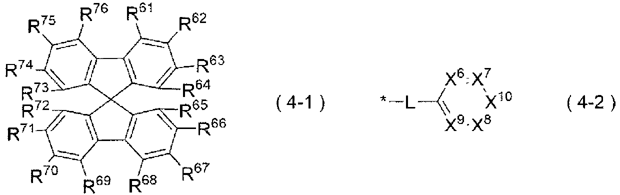 where
R⁶¹ through R⁷⁶ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, a heteroaryl group having 3 to 30 carbon atoms, and a group represented by formula (4-2), or form a ring by bonding of two adjacent groups thereof,
one of R⁶¹ through R⁷⁴ is a group represented by formula (4-2),
L represents a direct bond or an aromatic hydrocarbon group having 6 to 16 carbon atoms,
X⁶ through X¹⁰ each represent a nitrogen atom, C(CN), or CR⁷⁷,
among X⁹ and X¹⁰, X⁹ is C(CN) or X¹⁰ is a nitrogen atom or C(CN),
among X⁶ through X¹⁰, the number of nitrogen atoms is 0 or 1, the number of C(CN) is 2 or 3 in a case where the number of nitrogen atoms is 0, and the number of C(CN) is 2 in a case where the number of nitrogen atoms is 1,
each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, and an alkoxy group having 1 to 10 carbon atoms, and
R⁶¹ through R⁷⁷ and L have at least one substituent or have no substituent.

In the compound in accordance with aspect 29 of the present invention, it is preferable in the aspect 28 that: each R⁷⁷ is independently selected from a hydrogen atom, an alkyl group having 1 to 20 carbon atoms, and a cycloalkyl group having 3 to 20 carbon atoms.

In the compound in accordance with aspect 30 of the present invention, it is preferable in the aspect 28 or 29 that: R⁶¹ through R⁷⁶ are selected from a hydrogen atom, a cyano group, an alkyl group having 1 to 20 carbon atoms, a cycloalkyl group having 3 to 20 carbon atoms, an aromatic hydrocarbon group having 6 to 16 carbon atoms, a heteroaryl group having 3 to 16 carbon atoms, and a group represented by formula (4-2) or form an aromatic hydrocarbon ring by bonding of two adjacent groups thereof.

In the compound in accordance with aspect 31 of the present invention, it is preferable in one of the aspects 28 through 30 that: X¹⁰ is a nitrogen atom or C(CN); X⁷ and X⁸ are C(CN) or CR⁷⁷; and X⁶ and X⁹ are CR⁷⁷.

In the compound in accordance with aspect 32 of the present invention, it is preferable in one of the aspects 28 through 31 that: a LUMO level is -2.2 eV or less, the LUMO level being a quantum calculation value which is calculated by density functional theory (DFT) using a B3LYP functional and a 6-31G(d) basis function.

In the compound in accordance with aspect 33 of the present invention, it is preferable in one of the aspects 28 through 32 that: a glass transition temperature is 140°C or higher.

The photoelectric conversion element material in accordance with aspect 34 of the present invention preferably contains the compound in accordance with one of the aspects 28 through 33.

The photoelectric conversion element material in accordance with aspect 35 of the present invention is, in the aspect 34, preferably a hole blocking material.

The photoelectric conversion element in accordance with aspect 36 of the present invention preferably includes a layer containing the photoelectric conversion element material in accordance with the aspect 34.

The photoelectric conversion element in accordance with aspect 37 of the present invention can be, in the aspect 36, an imaging element.

The present invention is not limited to the embodiments, but can be altered by a skilled person in the art within the scope of the claims. The present invention also encompasses, in its technical scope, any embodiment derived by combining technical means disclosed in differing embodiments.

### Examples

The present invention will be described in more detail based on Examples below. Note, however, that the present invention should not be limitedly interpreted by those Examples to any extent.

### Synthesis example 1

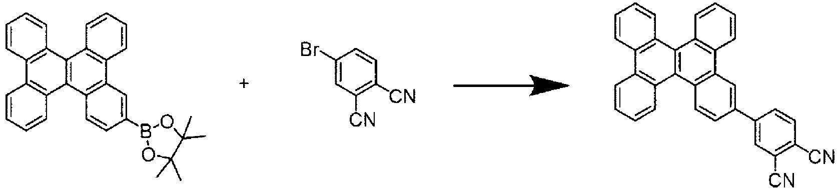

Under an argon airflow, (4,4,5,5-tetramethyl-2-(dibenzo[g,p]chrysen-3-yl)-1,3,2-dioxaborolane (2.2 g), 4-bromophthalonitrile (950 mg), and tetrakis(triphenylphosphine)palladium (168 mg) were suspended in 48 ml of THF, 2N of a tripotassium phosphate aqueous solution (7.3 ml) was added, and a resultant mixture was refluxed for 17 hours. A reaction solution was cooled to room temperature. Then, water was added and a resultant solid was collected by filtration, and thus 2.1 g of a yellow solid was obtained. The obtained yellow solid was recrystallized with 120 ml of toluene. Thus, an intended compound (B408) was obtained (1.7 g, yield: 78%).

Identification of the obtained compound (B408) was carried out by 1H-NMR.

1H-NMR (CDCl3) δ (ppm): 8.88 (d, J = 1.9 Hz, 1H), 8.83 (d, J = 9.3 Hz, 1H), 8.77-8.71 (m, 5H), 8.65 (d, J = 8.2 Hz, 1H), 8.26 (d, J = 1.4 Hz, 1H), 8.16 (dd, J = 8.5, 2.0 Hz, 1H), 7.96 (d, J = 8.3 Hz, 1H), 7.82 (dd, J = 8.7, 1.7 Hz, 1H), 7.77-7.65 (m, 6H).

### Synthesis example 2

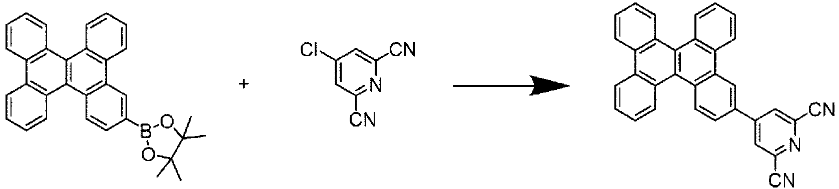

Under an argon airflow, (4,4,5,5-tetramethyl-2-(dibenzo[g,p]chrysen-3-yl)-1,3,2-dioxaborolane (2.0 g), 4-chloropyridine-2,6-dicarbonitrile (790 mg), palladium acetate (30 mg), and dicyclohexyl(2',4',6'-triisopropyl-(1,1'-biphenyl)-2-yl)phosphane (126 mg) were suspended in 44 ml of THF, 2N of a tripotassium phosphate aqueous solution (6.6 ml) was added, and a resultant mixture was refluxed for 6 hours. A reaction solution was cooled to room temperature. Then, water was added and a resultant solid was collected by filtration, and thus 2.1 g of a yellow solid was obtained. The obtained yellow solid was cleaned with hexane and methanol. Thus, an intended compound (B395) was obtained (2.0 g, yield: 100%).

Identification of the obtained compound (B395) was carried out by 1H-NMR.

1H-NMR (CDCl3) δ (ppm): 8.94 (d, J = 1.9 Hz, 1H), 8.86 (d, J = 8.7 Hz, 1H), 8.78-8.71 (m, 5H), 8.61 (d, J = 7.8 Hz, 1H), 8.29 (S, 2H), 8.84 (dd, J = 8.8, 2.5 Hz, 1H), 7.80-7.67 (m, 6H).

### Synthesis example 3

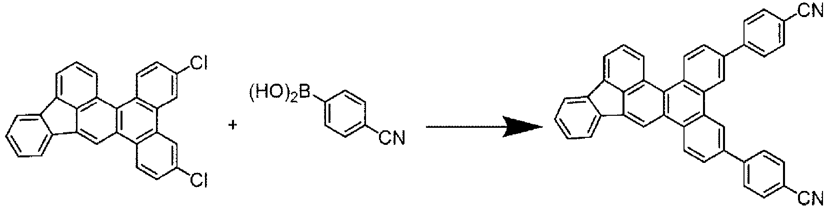

Under an argon airflow, 3,6-dichloro-benzo[g]indeno[1,2,3-qr]chrysene (1.7 g), 4-cyanophenylboronic acid (1.2 g), palladium acetate (27 mg), and dicyclohexyl(2',4',6'-triisopropyl-(1,1'-biphenyl)-2-yl)phosphane (114 mg) were suspended in 46 ml of THF, 2N of a tripotassium phosphate aqueous solution (6.0 ml) was added, and a resultant mixture was refluxed for 17 hours. A reaction solution was cooled to room temperature. Then, water was added and a resultant solid was collected by filtration, and thus 2.2 g of a yellow solid was obtained. The obtained yellow solid was recrystallized with 120 ml of toluene. Thus, an intended compound (B499) was obtained (2.0 g, yield: 89%).

Identification of the obtained compound (B499) was carried out by 1H-NMR.

1H-NMR (CDCl3) δ (ppm): 9.12 (s, 1H), 9.05 (d, J = 8.4 Hz, 1H), 8.93 (d, J = 5.1 Hz, 1H), 8.91 (dd, J = 5.3, 1.9 Hz, 2H), 8.89 (d, J = 8.6 Hz, 1H), 8.12-8.10 (m, 1H), 8.05 (d, J = 7.0 Hz, 1H), 8.00-7.91 (m, 7H), 7.87-7.82 (m, 5H), 7.50-7.48 (m, 2H).

### Synthesis example 4

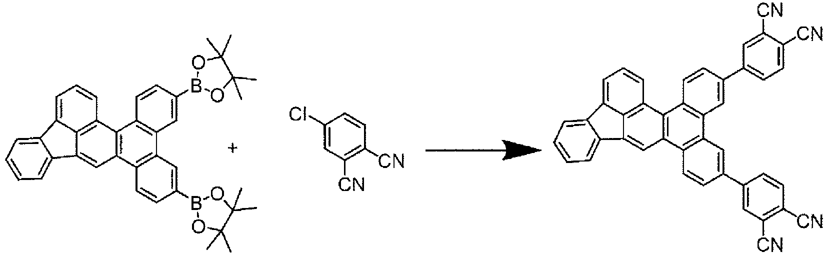

Under an argon airflow, 3,6-bis(4,4,5,5-tetramethyl-1,3,2-dioxaborolan-2-yl)-benzo[g]indeno[1,2,3-qr]chrysene (6.7 g), 4-bromophthalonitrile (4.6 g), and tetrakis(triphenylphosphine)palladium (380 mg) were suspended in 440 ml of THF, 2N of a tripotassium phosphate aqueous solution (33 ml) was added, and a resultant mixture was refluxed for 4 hours. A reaction solution was cooled to room temperature. Then, water was added and a resultant solid was collected by filtration, and thus 7.8 g of a yellow solid was obtained. The obtained yellow solid was cleaned with chlorobenzene. Thus, an intended compound (B509) was obtained (5.5 g, yield: 83%).

Identification of the obtained compound (B509) was carried out by 1H-NMR.

1H-NMR (DMSO) δ (ppm): 9.74 (s, 2H), 9.54 (d, J = 7.3 Hz, 2H), 9.33 (d, J = 9.0 Hz, 1H), 9.11 (d, J = 9.0 Hz, 1H), 8.95 (dd, J = 4.8 Hz, 1.6 Hz, 2H), 8.87 (d, J = 8.9 Hz, 1H), 8.70 (dd, J = 8.6 Hz, 2.0 Hz, 1H), 8.65 (dd, J = 8.3 Hz, 1.8 Hz, 1H), 8.45 (d, J = 6.5 Hz, 1H), 8.40 (dd, J = 8.0 Hz, 1.7 Hz, 2H), 8.36-8.30 (m, 2H), 8.16 (d, J = 6.6 Hz, 1H), 7.98 (t, J = 7.8 Hz, 1H), 7.55 (t, J = 4.0 Hz, 2H).

### Synthesis example 5

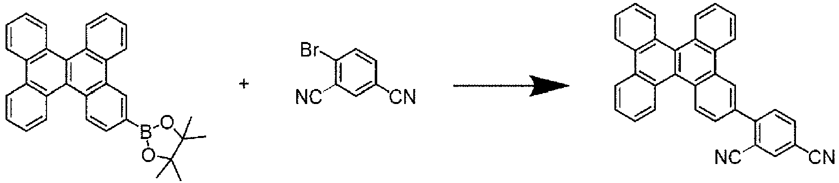

Under an argon airflow, (4,4,5,5-tetramethyl-2-(dibenzo[g,p]chrysen-3-yl)-1,3,2-dioxaborolane (2.2 g), 4-bromophthalonitrile (910 mg), and tetrakis(triphenylphosphine)palladium (150 mg) were suspended in 44 ml of THF, 2N of a tripotassium phosphate aqueous solution (6.6 ml) was added, and a resultant mixture was refluxed for 5 hours. A reaction solution was cooled to room temperature. Then, water was added and a resultant solid was collected by filtration, and thus 2.0 g of a yellow solid was obtained. The obtained yellow solid was recrystallized with toluene. Thus, an intended compound (B426) was obtained (1.5 g, yield: 75%).

Identification of the obtained compound (B426) was carried out by 1H-NMR.

1H-NMR (CDCl3) δ (ppm): 8.86 (d, J = 2.1 Hz, 1H), 8.81 (d, J = 8.7 Hz, 1H), 8.72-8.67 (m, 5H), 8.65 (d, J = 8.4 Hz, 1H), 8.11 (d, J = 1.7 Hz, 1H), 7.94 (dd, J = 8.3, 1.6 Hz, 1H), 7.81 (d, J = 8.3 Hz, 1H), 7.77 (dd, J = 8.3, 2.2 Hz, 1H), 7.73-7.64 (m, 6H).

### Synthesis example 6

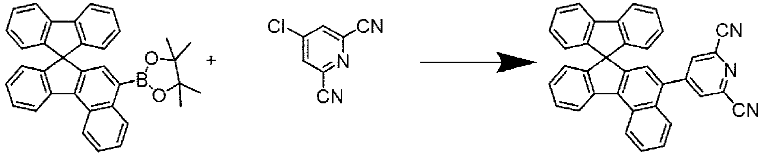

Under an argon airflow, (4,4,5,5-tetramethyl-2-(spiro(benzo[c]fluorene-7,9'-fluoren)-5-yl)-1,3,2-dioxaborolane (1.0 g), 4-chloropyridine-2,6-dicarbonitrile (315 mg), palladium acetate (13 mg), and dicyclohexyl(2',4',6'-triisopropyl-(1,1'-biphenyl)-2-yl)phosphane (58 mg) were suspended in 20 ml of THF, 2N of a tripotassium phosphate aqueous solution (3.0 ml) was added, and a resultant mixture was refluxed for 4 hours. A reaction solution was cooled to room temperature. Then, water was added and a resultant solid was collected by filtration, and thus 0.95 g of a yellow solid was obtained. The obtained yellow solid was purified by silica gel column chromatography, and thus an intended compound (B622) was obtained (0.7 g, yield: 70%).

Identification of the obtained compound (B622) was carried out by 1H-NMR.

1H-NMR (DMSO) δ (ppm): 8.98 (s, 2H), 8.60 (d, J = 8.3 Hz, 1H), 7.94 (t, J = 5.9 Hz, 2H), 7.70 (t, J = 7.5 Hz, 1H), 7.51 (t, J = 7.6 Hz, 1H), 7.40-7.11 (m, 8H), 7.03 (t, J = 7.7 Hz, 1H), 6.71 (d, J = 7.3 Hz, 1H), 6.67 (d, J = 6.7, 1H), 6.57 (d, J = 7.3 Hz, 1H).

### Synthesis example 7

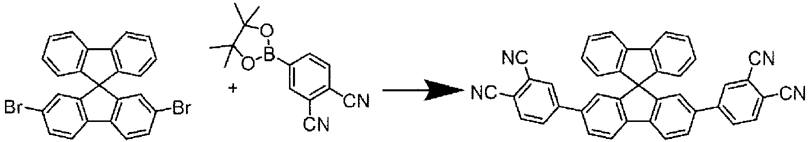

Under an argon airflow, 2,7-dibromo-9,9'-spirobifluorene (1.5 g), 4-(4,4,5,5-tetramethyl-1,3,2-dioxaborolan-2-yl)phthalonitrile (844 mg), and tetrakis(triphenylphosphine)palladium (110 mg) were suspended in 30 ml of THF, 2N of a tripotassium phosphate aqueous solution (4.7 ml) was added, and a resultant mixture was refluxed for 5 hours. A reaction solution was cooled to room temperature. Then, water was added and a resultant solid was collected by filtration, and thus 1.81 g of a yellow solid was obtained. The obtained yellow solid was recrystallized with 180 ml of chlorobenzene. Thus, an intended compound (B577) was obtained (1.1 g, yield: 61%).

Identification of the obtained compound (B577) was carried out by 1H-NMR.

1H-NMR (DMSO) δ (ppm): 8.35 (s, 2H), 8.32 (d, J = 7.8 Hz, 2H), 8.08-8.02 (m, 6H), 7.98 (dd, J = 7.8 Hz, 1.6 Hz, 2H), 7.44 (t, J = 7.6 Hz, 2H), 7.15 (t, J = 7.6 Hz, 2H), 7.09 (s, 2H), 7.70 (d, J = 7.2 Hz, 2H).

### Synthesis example 8

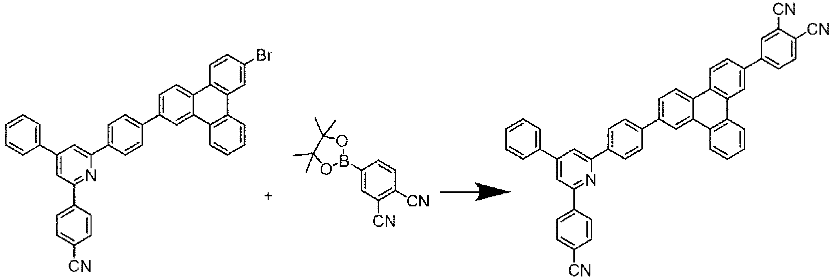

Under an argon airflow, 2-(4-(2-bromotriphenylen-7-yl)phenyl)-6-(4-cyanophenyl)-4-phenylpyridine (1.0 g), 4-(4,4,5,5-tetramethyl-1,3,2-dioxaborolan-2-yl)phthalonitrile (610 mg), palladium acetate (7.2 mg), and dicyclohexyl(2',4',6'-triisopropyl-(1,1'-biphenyl)-2-yl)phosphane (30 mg) were suspended in 16 ml of THF, 2N of a tripotassium phosphate aqueous solution (2.4 ml) was added, and a resultant mixture was refluxed for 4 hours. A reaction solution was cooled to room temperature. Then, water was added and a resultant solid was collected by filtration, and thus 1.3 g of a solid was obtained. The obtained solid was purified by silica gel column chromatography, and thus an intended compound (B104) was obtained (0.81 g, yield: 73%).

Identification of the obtained compound (B104) was carried out by 1H-NMR.

1H-NMR (DMSO) δ (ppm): 8.96 (s, 1H), 8.86-8.77 (m, 4H), 8.37 (dd, J = 8.4 Hz, 3.4 Hz, 4H), 8.24 (d, J = 1.8 Hz, 1H), 8.16 (dd, J = 8.1 Hz, 1.8 Hz, 1H), 8.05-7.95 (m, 6H), 7.88 (d, J = 8.1 Hz, 1H), 7.83 (d, J = 8.7 Hz, 1H), 7.78-7.75 (m, 4H), 7.59-7.50 (m, 4H).

### <Glass transition temperature, LUMO level>

The glass transition temperatures of the compounds (B408), (B395), (B499), (B509), (B426), (B622), (B577), and (B104) in synthesis examples 1 through 8, a comparative compound 1 described in Patent Literature 1, and a comparative compound 2 described in Patent Literature 2 were measured at a sweep rate of 10°C/min using a differential scanning calorimeter (DSC702 available from Hitachi High-Technologies Corporation) and using an aluminum pan. For the LUMO level, optimization of the molecular structure and calculation of the LUMO level were carried out using Gaussian 16 software and using density functional theory (DFT) under calculation conditions of a B3LYP functional and a 6-31G(d) basis function. The obtained results of glass transition temperature and LUMO level (Calc.LUMO) are indicated in Table 1.

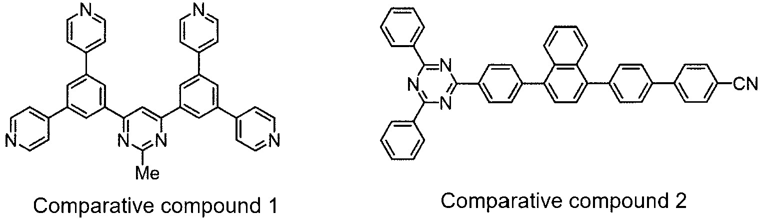

**[Table 1]**

| | Compound | Tg | calc. LUMO |
|---|---|---|---|
| Synthesis example 1 | B408 | 142 | -2.4 |
| Synthesis example 2 | B395 | 165 | -2.5 |
| Synthesis example 3 | B499 | 154 | -2.4 |
| Synthesis example 4 | B509 | N. D. (>154°C) | -2.8 |
| Synthesis example 5 | B426 | 148 | -2.4 |
| Synthesis example 6 | B622 | N. D. (>150°C) | -2.4 |
| Synthesis example 7 | B577 | 217 | -2.7 |
| Synthesis example 8 | B104 | 176 | -2.5 |
| - | Comparative compound 1 | N. D. (<130°C) | -2.2 |
| - | Comparative compound 2 | 116 | -2.0 |

### <Element example 1 (see Fig. 1)>

As illustrated in Fig. 1, the imaging element 100 was prepared as a photoelectric conversion element having a laminated structure constituted by the first electrode 1, the hole blocking layer 2, the photoelectric conversion layer 3, the electron blocking layer 4, the hole transport layer 5, and the second electrode 6, and the dark current, external quantum efficiency, and responsiveness of the imaging element were evaluated.

### (Preparation of first electrode 1)

As a substrate having a surface on which the first electrode is provided, a glass substrate was prepared which was provided with an ITO transparent electrode in which an indium-tin oxide (ITO) film (film thickness: 110 nm) having a width of 2 mm was patterned in stripes. Then, the substrate was cleaned with isopropyl alcohol, and a surface treatment was carried out with UV ozone cleaning.

### (Preparation of vacuum deposition)

Vacuum deposition of each layer was carried out by a vacuum deposition method on the substrate which had been subjected to the surface treatment after the cleaning, and thus a lamination of the layers was formed.

First, the glass substrate was introduced into a vacuum deposition tank, and pressure was reduced to 7.0×10⁻⁵ Pa. Then, in the following order, layers were prepared in accordance with film formation conditions of the respective layers.

### (Preparation of hole blocking layer 2)

The sublimation-purified compound (B408) was formed into a film of 10 nm at a rate of 0.03 nm/sec, and thus a hole blocking layer 2 was prepared.

### (Preparation of photoelectric conversion layer (light reception layer) 3)

N,N-dimethylquinacridone and C60 were formed into a film of 120 nm at a proportion of 4:1 (mass ratio), and thus a photoelectric conversion layer 3 was prepared. The film formation rate was 0.15 nm/sec.

### (Preparation of electron blocking layer 4)

The compound (ic-3) was formed into a film of 10 nm at a rate of 0.10 nm/sec, and thus an electron blocking layer 4 was prepared. Note that (ic-3) was synthesized by a method disclosed in Japanese Patent Application Publication, Tokukai, No. 2018-193371.

### (Preparation of hole transport layer 5)

The compound 2,3,6,7,10,11-hexacyano-1,4,5,8,9,12-hexaazatriphenylene (HATCN) was formed into a film of 10 nm at a rate of 0.10 nm/sec, and thus a hole transport layer 5 was prepared.

### (Preparation of second electrode 6)

Lastly, a metal mask was disposed so as to extend straight with the ITO stripes on the substrate, and a second electrode 6, which was an upper electrode, was formed as a film. Specifically, silver was formed into a film of 80 nm at a rate of 0.1 nm/sec, and the second electrode 6, which was an upper electrode, was prepared.

Thus, the photoelectric conversion element 100 for imaging having an area of 4 mm² as illustrated in Fig. 1 was prepared. Note that each of the film thicknesses was measured by a stylus film thickness meter (DEKTAK, available from Bruker).

In addition, the element was sealed within a nitrogen atmosphere glove box having an oxygen and water concentration of 1 ppm or less. The sealing was carried out using a glass sealing cap, the film-formed substrate (element), and a bisphenol-F epoxy resin (available from Nagase ChemteX Corporation).

The electric current in the dark (dark current), external quantum efficiency, and response time when a voltage of 2.6 V was applied to the imaging element prepared as described above were evaluated. Measurement of the dark current was evaluated using a sources measure unit 2636B available from Keithley Instruments. A solar cell spectral sensitivity measurement device (available from SOMA OPT LTD.) was used to measure the external quantum efficiency. The measurement was carried out with irradiation light having a wavelength of 560 nm and intensity of 50 µW/cm². As the response time, a time from when irradiation with a light pulse was carried out to when, after the irradiation, the current value returned to the value before the irradiation was measured.

Note that the dark current, external quantum efficiency, and response time are relative values where the result in element comparative example 1 is set to be a reference value (1.0). A lower dark current indicates that performance is more excellent, higher external quantum efficiency indicates that performance is more excellent, and a shorter response time indicates that performance is more excellent. The obtained measurement results are shown in Table 1.

### Element examples 2 through 7, element comparative examples 1 and 2

Imaging photoelectric conversion elements were prepared with a method similar to that of element example 1 except that, in element example 1, the compound (B395), the compound (B499), the compound (B509), the compound (B426), the compound (B577), the compound (B104), and the comparative compound 1 were used in order, respectively, instead of the compound (B408), and evaluation was carried out. The obtained measurement results are shown in Table 1.

**[Table 2]**

| | Compound | Dark current | External quantum efficiency | Responsiveness |
|---|---|---|---|---|
| Element example 1 | B408 | 0.93 | 1.1 | 0.21 |
| Element example 2 | B395 | 0.86 | 1.06 | 0.23 |
| Element example 3 | B499 | | 1.07 | 0.24 |
| Element example 4 | B509 | 0.84 | 1.06 | 0.23 |
| Element example 5 | B426 | 0.50 | 1.04 | 0.23 |
| Element example 6 | B577 | 0.68 | 1.03 | 0.35 |
| Element example 7 | B104 | 0.61 | 1.07 | 0.25 |
| Element comparative example 1 | Comparative compound 1 | 1 | 1 | 1 |

The results in Table 1 show that, by forming a layer using, as a material for an imaging element or a photoelectric conversion element, the compound represented by formula (1) or formula (3) or formula (4-1), it is possible to achieve excellent dark current, external quantum efficiency, and responsiveness as compared with the case where a layer is formed using the comparative example compound.

### Industrial Applicability

The imaging element including the photoelectric conversion element in accordance with an aspect of the present invention can be applied, for example, to imaging elements of a digital camera and a digital video camera, to an imaging element built in a mobile phone or the like, to an image input device of a driving assistance system, and the like.

### Reference Signs List

1: First electrode
2: Hole blocking layer
3: Photoelectric conversion layer (light reception layer)
4: Electron blocking layer
5: Hole transport layer
6: Second electrode
10: Organic layer
100: Imaging element (photoelectric conversion element)

This application is a divisional application of European patent application no. 23900761.0 (the "parent application"). The claims of the parent application are repeated below as "items" in the present specification and shall form part of the content of the description of this divisional application as filed:

### [Item 1]

A photoelectric conversion element comprising a layer containing a photoelectric conversion element material,
the photoelectric conversion element material being represented by formula (1) below: 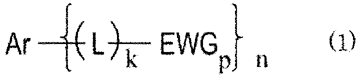
where
Ar represents a condensed ring aromatic hydrocarbon group having 16 to 40 carbon atoms,
EWG represents an electron-withdrawing group,
L represents an aromatic hydrocarbon group having 6 to 30 carbon atoms,
n represents an integer of 1 to 8,
k represents an integer of 0 to 2,
p represents an integer of 1 to 8,
in a case where k is 0, p is 1,
in a case where there are two or more EWG, the two or more EWG are identical or different electron-withdrawing groups, and
each of Ar, L, and EWG has at least one substituent or has no substituent.

### [Item 2]

The photoelectric conversion element as set forth in item 1, wherein: the EWG is selected from a cyano group, a fluorine atom, a fluoroalkyl group, and a heteroaryl group that has 3 to 30 carbon atoms and that includes a nitrogen atom.

### [Item 3]

The photoelectric conversion element as set forth in item 2, wherein:
the heteroaryl group having 3 to 30 carbon atoms and including a nitrogen atom has a substituent; and
at least one of the substituent is a group selected from a cyano group, a fluorine atom, and a fluoroalkyl group.

### [Item 4]

The photoelectric conversion element as set forth in item 1, wherein:
k is an integer of 0 or 1.

### [Item 5]

The photoelectric conversion element as set forth in item 1, wherein:
k is 1; and
the EWG is at least one group selected from a cyano group, a fluorine group, and a fluoroalkyl group.

### [Item 6]

The photoelectric conversion element as set forth in item 1, wherein:
Ar is a condensed ring aromatic hydrocarbon group which encompasses four or more 6-membered rings.

### [Item 7]

The photoelectric conversion element as set forth in item 1, wherein:
Ar is selected from (i) a condensed ring aromatic hydrocarbon group which is selected from a triphenylene group, a fluoranthene group, and a spirofluoren group and (ii) a condensed ring aromatic hydrocarbon group which encompasses a triphenylene group, a fluoranthene group, and a spirofluoren group.

### [Item 8]

The photoelectric conversion element as set forth in item 1, wherein:
Ar represents a condensed ring aromatic hydrocarbon group having 20 to 40 carbon atoms, the condensed ring aromatic hydrocarbon group having the at least one substituent or having no substituent.

### [Item 9]

The photoelectric conversion element as set forth in item 1, wherein:
Ar is represented by formulae (2-1), (2-2), and (2-3) below; 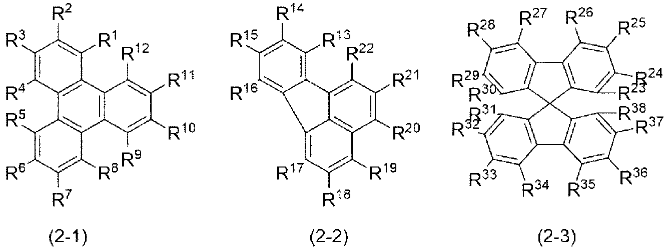
where
R¹ through R³⁸ are each independently selected from a hydrogen atom, an alkyl group having 1 to 20 carbon atoms, an alkenyl group having 1 to 20 carbon atoms, a cycloalkyl group having 1 to 20 carbon atoms, a bicycloalkyl group having 1 to 20 carbon atoms, a tricycloalkyl group having 1 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R¹ through R³⁸ form a ring by bonding of two adjacent groups thereof, and
R¹ through R³⁵ each independently has at least one substituent or has no substituent.

### [Item 10]

The photoelectric conversion element as set forth in item 1, wherein:
the photoelectric conversion element material has a LUMO level of -2.2 eV or less, the LUMO level being a quantum calculation value which is calculated by density functional theory (DFT) using a B3LYP functional and a 6-31G(d) basis function.

### [Item 11]

The photoelectric conversion element as set forth in item 1, wherein:
a glass transition temperature of the photoelectric conversion element material is 140°C or higher.

### [Item 12]

The photoelectric conversion element as set forth in item 1, wherein:
the layer containing the photoelectric conversion element material is a hole blocking layer.

### [Item 13]

The photoelectric conversion element as set forth in item 1, wherein said photoelectric conversion element is an imaging element.

### [Item 14]

A photoelectric conversion element material for forming a layer included in a photoelectric conversion element recited in any one of items 1 through 13,
said photoelectric conversion element material being represented by the formula (1).

### [Item 15]

The photoelectric conversion element material as set forth in item 14, wherein:
said photoelectric conversion element material is a photoelectric conversion element material for an imaging element.

### [Item 16]

The photoelectric conversion element material as set forth in item 14, wherein:
said photoelectric conversion element material is a hole blocking material.

### [Item 17]

The photoelectric conversion element as set forth in item 12, further comprising a fullerene-containing layer which is disposed between the hole blocking layer and an electrode.

### [Item 18]

A compound represented by formula (3) below: 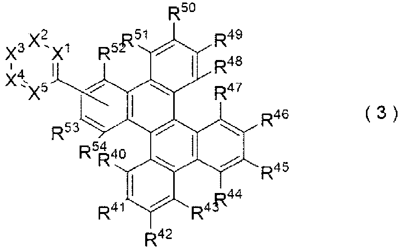 where
X¹ through X⁵ each represent a nitrogen atom or CR⁵⁵,
the number of nitrogen atoms in X¹ through X⁵ is an integer of 0 to 3,
R⁴⁰ through R⁵⁵ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 1 to 20 carbon atoms, a cycloalkyl group having 1 to 20 carbon atoms, a bicycloalkyl group having 1 to 20 carbon atoms, a tricycloalkyl group having 1 to 20 carbon atoms, an alkoxy group having 1 to 10 carbon atoms, an aromatic hydrocarbon group having 6 to 30 carbon atoms, and a heteroaryl group having 3 to 30 carbon atoms, or
some of R⁴⁰ through R⁵⁵ form a ring by bonding of two adjacent groups thereof,
at least one R⁵⁵ is selected from a cyano group, a halogen atom, and an alkyl halide group,
in a case where X¹ through X⁵ are all CR⁵⁵, two or more of R⁵⁵ are selected from a cyano group, a halogen atom, and an alkyl halide group, and
R⁴⁰ through R⁵⁵ each independently have further at least one substituent or have no substituent.

### [Item 19]

The compound as set forth in item 18, wherein:
each R⁵⁵ is independently selected from a hydrogen atom, a cyano group, a halogen atom, an alkyl halide group, an alkyl group having 1 to 20 carbon atoms, and a cycloalkyl group having 1 to 20 carbon atoms.

### [Item 20]

The compound as set forth in item 18, wherein:
one of R⁵⁵ is a cyano group.

### [Item 21]

The compound as set forth in item 18, wherein:
the halogen atom is a fluorine atom; and
the aromatic hydrocarbon group having 6 to 30 carbon atoms is substituted by a group selected from a cyano group, a fluorine group, and a fluoroalkyl group.

### [Item 22]

The compound as set forth in item 18, wherein:
a LUMO level is -2.2 eV or less, the LUMO level being a quantum calculation value which is calculated by density functional theory (DFT) using a B3LYP functional and a 6-31G(d) basis function.

### [Item 23]

The compound as set forth in item 18, wherein:
a glass transition temperature is 140°C or higher.

### [Item 24]

A photoelectric conversion element material, comprising a compound recited in any one of items 18 through 23.

### [Item 25]

The photoelectric conversion element material as set forth in item 24, wherein:
said photoelectric conversion element material is a hole blocking material.

### [Item 26]

A photoelectric conversion element, comprising a layer which contains a photoelectric conversion element material recited in item 24.

### [Item 27]

The photoelectric conversion element as set forth in item 26, wherein said photoelectric conversion element is an imaging element.

### [Item 28]

A compound represented by formula (4-1) below: 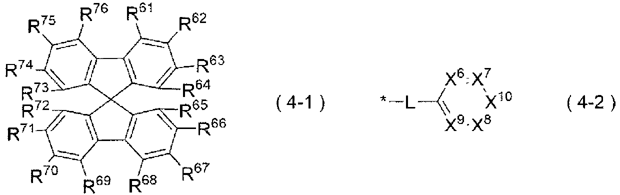 where
R⁶¹ through R⁷⁶ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 1 to 20 carbon atoms, a cycloalkyl group having 1 to 20 carbon atoms, a bicycloalkyl group having 1 to 20 carbon atoms, a tricycloalkyl group having 1 to 20 carbon atoms, an alkoxy group having 1 to 10 carbon atoms, an aromatic hydrocarbon group having 6 to 30 carbon atoms, a heteroaryl group having 3 to 30 carbon atoms, and a group represented by formula (4-2), or form a ring by bonding of two adjacent groups thereof,
one of R⁶¹ through R⁷⁶ is a group represented by formula (4-2),
L represents a direct bond or an aromatic hydrocarbon group having 6 to 16 carbon atoms,
X⁶ through X¹⁰ each represent a nitrogen atom, C(CN), or CR⁷⁷,
among X⁹ and X¹⁰, X⁹ is C(CN) or X¹⁰ is a nitrogen atom or C(CN),
among X⁶ through X¹⁰, the number of nitrogen atoms is 0 or 1, the number of C(CN) is 2 or 3 in a case where the number of nitrogen atoms is 0, and the number of C(CN) is 2 in a case where the number of nitrogen atoms is 1,
each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 1 to 20 carbon atoms, a cycloalkyl group having 1 to 20 carbon atoms, a bicycloalkyl group having 1 to 20 carbon atoms, a tricycloalkyl group having 1 to 20 carbon atoms, and an alkoxy group having 1 to 10 carbon atoms, and
R⁶¹ through R⁷⁷ and L have at least one substituent or have no substituent.

### [Item 29]

The compound as set forth in item 28, wherein:
each R⁷⁷ is independently selected from a hydrogen atom, an alkyl group having 1 to 20 carbon atoms, and a cycloalkyl group having 1 to 20 carbon atoms.

### [Item 30]

The compound as set forth in item 28, wherein:
R⁶¹ through R⁷⁶ are selected from a hydrogen atom, a cyano group, an alkyl group having 1 to 20 carbon atoms, a cycloalkyl group having 1 to 20 carbon atoms, an aromatic hydrocarbon group having 6 to 16 carbon atoms, a heteroaryl group having 3 to 16 carbon atoms, and a group represented by formula (4-2) or form an aromatic hydrocarbon ring by bonding of two adjacent groups thereof.

### [Item 31]

The compound as set forth in item 28, wherein:
X¹⁰ is a nitrogen atom or C(CN);
X⁷ and X⁸ are C(CN) or CR⁷⁷; and
X⁶ and X⁹ are CR⁷⁷.

### [Item 32]

The compound as set forth in item 28, wherein:
a LUMO level is -2.2 eV or less, the LUMO level being a quantum calculation value which is calculated by density functional theory (DFT) using a B3LYP functional and a 6-31G(d) basis function.

### [Item 33]

The compound as set forth in item 28, wherein:
a glass transition temperature is 140°C or higher.

### [Item 34]

A photoelectric conversion element material, comprising a compound recited in any one of items 28 through 33.

### [Item 35]

The photoelectric conversion element material as set forth in item 34, wherein:
said photoelectric conversion element material is a hole blocking material.

### [Item 36]

A photoelectric conversion element, comprising a layer which contains a photoelectric conversion element material recited in item 34.

### [Item 37]

The photoelectric conversion element as set forth in item 36, wherein said photoelectric conversion element is an imaging element.

## Claims

1. A compound represented by formula (3) or (4-1):
the compound represented by formula (3) below: 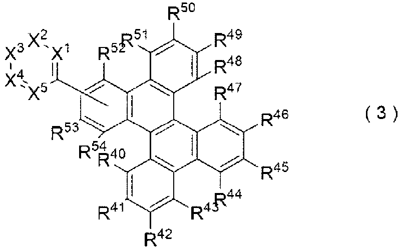
where
X¹ through X⁵ each represent a nitrogen atom or CR⁵⁵,
the number of nitrogen atoms in X¹ through X⁵ is an integer of 0 to 3,
R⁴⁰ through R⁵⁵ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, and a heteroaryl group having 3 to 30 carbon atoms, the halogen atom is preferably a fluorine atom and the aromatic hydrocarbon group having 6 to 30 carbon atoms might be substituted by a group selected from a cyano group, a fluorine group, and a fluoroalkyl group; or
some of R⁴⁰ through R⁵⁵ form a ring by bonding of two adjacent groups thereof,
at least one R⁵⁵ is selected from a cyano group, a halogen atom, and an alkyl halide group,
in a case where X¹ through X⁵ are all CR⁵⁵, two or more of R⁵⁵ are selected from a cyano group, a halogen atom, and an alkyl halide group, and
R⁴⁰ through R⁵⁵ each independently have further at least one substituent or have no substituent: and
the compound represented by formula (4-1) below: 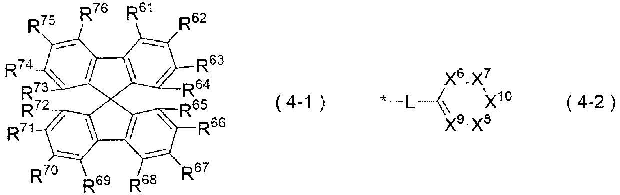
where
R⁶¹ through R⁷⁶ are each independently selected from a hydrogen atom, a cyano group, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, an alkoxy group having 1 to 10 carbon atoms, an aromatic hydrocarbon group having 6 to 30 carbon atoms, a heteroaryl group having 3 to 30 carbon atoms, and a group represented by formula (4-2), or form a ring by bonding of two adjacent groups thereof,
one of R⁶¹ through R⁷⁶ is a group represented by formula (4-2),
L represents a direct bond or an aromatic hydrocarbon group having 6 to 16 carbon atoms,
X⁶ through X¹⁰ each represent a nitrogen atom, C(CN), or CR⁷⁷,
among X⁹ and X¹⁰, X⁹ is C(CN) or X¹⁰ is a nitrogen atom or C(CN),
among X⁶ through X¹⁰, the number of nitrogen atoms is 0 or 1, the number of C(CN) is 2 or 3 in a case where the number of nitrogen atoms is 0, and the number of C(CN) is 2 in a case where the number of nitrogen atoms is 1,
each R⁷⁷ is independently selected from a hydrogen atom, a nitro group, a halogen atom, an alkyl halide group, an acyl group, a sulfonyl group, a phosphoryl group, an alkyl group having 1 to 20 carbon atoms, an alkenyl group having 2 to 20 carbon atoms, a cycloalkyl group having 3 to 20 carbon atoms, a bicycloalkyl group having 4 to 20 carbon atoms, a tricycloalkyl group having 5 to 20 carbon atoms, and an alkoxy group having 1 to 10 carbon atoms, and
R⁶¹ through R⁷⁷ and L have at least one substituent or have no substituent.

2. The compound as set forth in claim 1, wherein:
each R⁵⁵ is independently selected from a hydrogen atom, a cyano group, a halogen atom, an alkyl halide group, an alkyl group having 1 to 20 carbon atoms, and a cycloalkyl group having 3 to 20 carbon atoms.

3. The compound as set forth in claim 1 or 2, wherein:
one of R⁵⁵ is a cyano group.

4. The compound as set forth in claim 1, wherein:
each R⁷⁷ is independently selected from a hydrogen atom, an alkyl group having 1 to 20 carbon atoms, and a cycloalkyl group having 3 to 20 carbon atoms.

5. The compound as set forth in claim 1 or 4, wherein:
R⁶¹ through R⁷⁶ are selected from a hydrogen atom, a cyano group, an alkyl group having 1 to 20 carbon atoms, a cycloalkyl group having 3 to 20 carbon atoms, an aromatic hydrocarbon group having 6 to 16 carbon atoms, a heteroaryl group having 3 to 16 carbon atoms, and a group represented by formula (4-2) or form an aromatic hydrocarbon ring by bonding of two adjacent groups thereof.

6. The compound as set forth in claim 1, 4 or 5, wherein:
X¹⁰ is a nitrogen atom or C(CN);
X⁷ and X⁸ are C(CN) or CR⁷⁷; and
X⁶ and X⁹ are CR⁷⁷.

7. The compound as set forth in any one of claims 1 through 6, wherein:
a LUMO level is -2.2 eV or less, the LUMO level being a quantum calculation value which is calculated by density functional theory (DFT) using a B3LYP functional and a 6-31G(d) basis function.

8. The compound as set forth in any one of claims 1 through 7, wherein:
a glass transition temperature is 140°C or higher.

9. A photoelectric conversion element material, comprising a compound recited in any one of claims 1 through 8.

10. The photoelectric conversion element material as set forth in claim 9, wherein:
said photoelectric conversion element material is a hole blocking material.

11. A photoelectric conversion element, comprising a layer which contains a photoelectric conversion element material recited in claim 10.

12. The photoelectric conversion element as set forth in claim 11, wherein said photoelectric conversion element is an imaging element.
